(19) Europäisches Patentamt
European Patent Office
Office européen des brevets

(11) Numéro de publication: **0 064 452 B1**

(12) # FASCICULE DE BREVET EUROPEEN

(45) Date de publication du fascicule du brevet: **17.04.85**

(51) Int. Cl.⁴: **C 12 P 19/00**, C 12 N 9/24, C 08 B 37/10

(21) Numéro de dépôt: **82400739.7**

(22) Date de dépôt: **26.04.82**

(54) **Oligosaccharides à chaînes courtes possédant des propriétés biologiques, leur préparation et leurs applications en tant que médicaments.**

(30) Priorite 29.04.81 FR 8108604

(43) Date de publication de la demande:
**10.11.82 Bulletin 82/45**

(45) Mention de la délivrance du brevet:
**17.04.85 Bulletin 85/16**

(84) Etats contractants désignés:
**AT BE CH DE GB IT LI LU NL SE**

(56) Documents cités:
**EP - A - 0 027 089**

**BIOCHEMISTRY, vol. 11, no. 4, 1972, pages 563-568 A. LINKER et al.: "Isolation and Characterization of Oligosaccharides Obtained from Haparin by the Action of Heparinase"**

(73) Titulaire: **CHOAY S.A., 48, Avenue Théophile-Gautier, F-75782 Paris Cédex 16 (FR)**

(72) Inventeur: **Lormeau, Jean-Claude, 1, rue Joseph Delattre, F-76150 Maromme (FR)**
Inventeur: **Choay, Jean, 21, Rue Saint-Guillaume, F-75007 Paris (FR)**
Inventeur: **Petitou, Maurice, 21, rue du Javelot Appart. 201, F-75645 Paris Cedex 13 (FR)**

(74) Mandataire: **Peaucelle, Chantal et al, Cabinet Plasseraud 84, rue d'Amsterdam, F-75009 Paris (FR)**

**EP 0 064 452 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

L'invention est relative à des oligosaccharides à chaînes courtes, possédant des propriétés biologiques leur permettant notamment de ne contrôler que certaines étapes de la coagulation sanguine.

Elle est relative à leur procédé d'obtention et à leurs applications en tant que principes actifs de médicaments.

Les inverteurs ont déjà décrit des oligosaccharides possédant des propriétés biologiques du type de celles évoquées ci-dessus.

Selon le procédé décrit dans la demande EP N° 0 027 089, les inventeurs ont notamment obtenu par dépolymérisation de l'héparine par voie chimique un mélange de dépolymérisation renfermant des octasaccharides et des oligosaccharides à chaînes plus courtes.

Le fractionnement de ce mélange a permis d'isoler des fractions d'octasaccharides de grande valeur répondant à la séquence A—B—C—D—E—F—G—H (désignée ci-après par l'abréviation A—H).

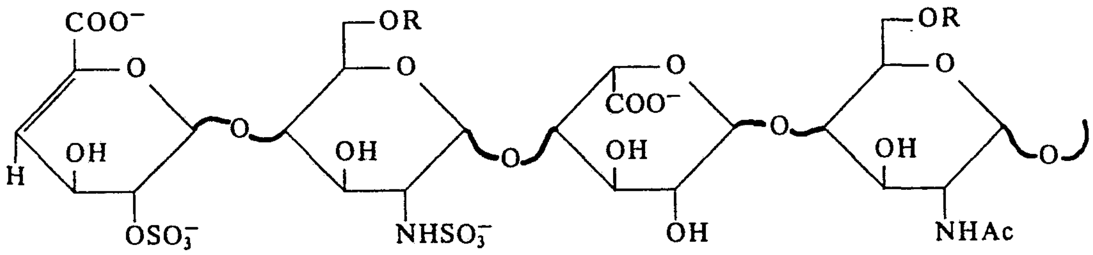

A B C D

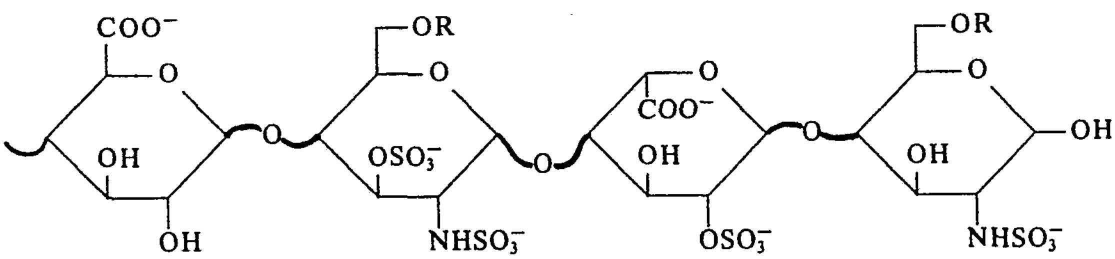

E F G H

dans laquelle R représente un atome d'hydrogène ou un groupe $-SO_3^-$.

Ces octasaccharides s'avèrent particulièrement précieux en raison de leur spécificité élevée vis-à-vis du facteur X activé ou facteur Xa du sang (mesurée selon le test Yin-Wessler) alors que leur activité sur la coagulation globale (mesurée selon le test USP ou APTT) est très faible.

Les références concernant la réalisation de ces différents tests seront données dans les exemples.

Dans l'article de A. LINKER et al paru dans Biochemistry, vol. 11. N° 4, 1972, pp. 563—568, on décrit un procédé de digestion d'héparine avec une héparinase. Ce procédé conduit cependant à des compositions d'oligosaccharides hétérogènes et dont l'activité spécifique n'est pas rapportée.

En poursuivant leurs travaux dans ce domaine, les inventeurs se sont intéressés, notamment, à l'obtention d'oligosaccharides à chaînes plus courtes que ces octasaccharides, mais possédant cependant un rapport avantageux de leur titre Yin-Wessler à leur titre USP ou APTT.

Ils ont ainsi été amenés à constater qu'en opérant dans des conditions bien déterminées, il était possible de raccourcir de manière sélective les chaînes actives des octasaccharides A—H et d'obtenir des compositions de grande homogénéité en oligosaccharides actifs à chaînes courtes.

L'invention a donc pour but de fournir de nouveaux oligosaccharides à chaînes plus courtes que les octasaccharides, possédant avantageusement au moins les propriétés biologiques de ces octasaccharides.

Elle vise également à fournir un procédé de mise en oeuvre aisée permettant d'éliminer de manière hautement sélective des motifs des octasaccharides A—H n'intervenant pas de manière essentielle dans l'activité biologique considérée de ces produits. Elle a également pour but de fournir des moyens d'obtention d'une enzyme particulièrement appropriée à la mise en oeuvre de ce procédé. Elle vise également à fournir des principes actifs de médicaments et les médicaments euxmêmes capables d'inhiber le facteur Xa quand il est présent dans le sang avec un degré de sélectivité élevé tout en possédant une très faible activité sur la coagulation totale, et utilisables, de ce fait, pour les traitements antithrombotiques sans risque hémorragique pour le patient.

Les compositions d'oligosaccharides de grande homogénéité, selon l'invention, sont caractérisées en ce qu'elles sont formées essentiellement d'hexasaccharides possédant la séquence C'DEFGH (désignée ci-après par l'abréviation C'—H).

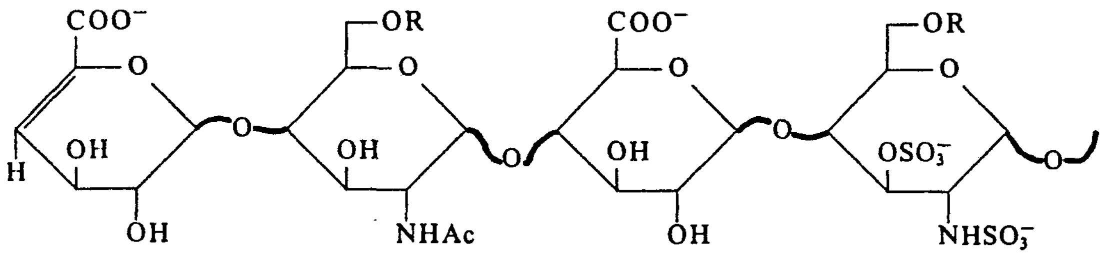

C' D E F

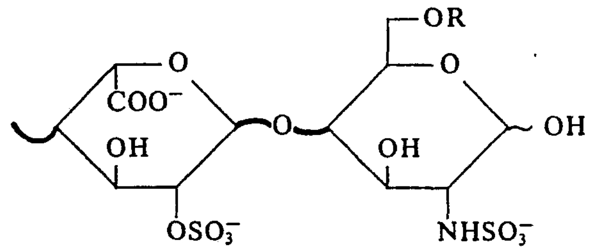

G H

dans laquelle R représente un atome d'hydrogène ou le groupe $-SO_3^-$.

Les hexasaccharides de l'invention sont caractérisés par une affinité élevée pour l'ATIII.

Ils sont, en outre, caractérisés par des spectres RMN comprenant, entre autres, un signal dans la région du carbone en position 2 des résidus N-sulfate-glucosamine, qui n'apparaît pas avec l'héparine. Ce signal peut être attribué à la présence d'un substituant sur l'atome d'oxygène en position 3, et plus particulièrement à un groupe 3-O-sulfate sur un résidu N-sulfate-D-glucosamine (motif F du schéma).

Les hexasaccharides de l'invention sont, de plus, caractérisés par des titres Yin-Wessler largement supérieurs à ceux de l'héparine. Plus spécialement, des compositions hexasaccharidiques de l'invention peuvent présenter des titres Yin-Wessler, supérieurs à 2000 µ/mg, pouvant même atteindre 2500 µ/mg environ. De manière avantageuse, leur titre APTT ou USP s'avère particulièrement faible, de l'ordre de 10, ce qui correspond à des compositions ayant des rapports de leur titre Yin-Wessler à leur titre USP ou APTT aussi élevés que 200, voire 250 environ.

Des compositions hexasaccharidiques avantageuses sont formées d'hexasaccharide C'—H dans lesquelles au moins un ou deux des groupes R représentent un groupe $-SO_3^-$.

L'invention vise également un procédé d'obtention des compositions hexasaccharidiques évoquées ci-dessus.

Selon ce procédé, on soumet, dans des conditions déterminées, des octasaccharides A—H, le cas échéant des compositions octasaccharidiques formées d'une majeure partie de ces octasaccharides A—H, à l'action d'une enzyme.

D'une amnière inattendue, il s'est avéré que les octasaacharides A—H, qui peuvent être eux-mêmes obtenus par action d'une enzyme sur les chaînes d'héparine, sont cependant encore dégradables par voie enzymatique. En opérant dans des conditions déterminées, il est alors possible d'éliminer des motifs n'intervenant pas de manière essentielle dans l'activité de ces produits, et ce avec une grande spécificité, ce qui permet d'obtenir des compositions hexasaccharidiques de grande homogénéité structurale.

Le procédé selon l'invention est donc caractérisé en ce qu'on met en contact les octasaccharides A—H évoqués ci-dessus, ou des compositions octasaccharidiques formées d'une majeure partie de ces octasaccharides, ayant un rapport élevé de leur titre Yin-Wessler à leur titre APTT ou USP, avec un agent enzymatique dans des conditions ajustées de manière à fragmenter ces octasaccharides de manière spécifique afin d'éliminer les motifs A—B n'intervenant pas de manière essentielle dans l'activité considérée, cette spécificité conduisant à l'obtention de mélanges formés pratiquement totalement d'hexasaccharides actifs.

Cette fragmentation spécifique est obtenue en mettant en oeuvre un agent enzymatique présentant une activité enzymatique d'au moins 90 000 unités, mis en oeuvre à raison de 0,25 à 1 mg par mg d'octasaccharides traités, de préférence, de l'ordre de 0,5 mg d'enzyme par mg d'octasaccharides. On sépare alors les hexasaccharides C'—H actifs du mélange de dégradation.

Selon une disposition préférée, la fragmentation est réalisée dans un milieu tampon renfermant du calcium.

Afin de disposer de compositions hexasaccharidiques hautement homogènes en hexasaccharides C'—H, on a avantageusement recours à un traitement permettant de séparer du mélange, dégradation au moins la majeure partie des hexasaccharides C'—H actifs.

Un traitement approprié comporte un fractionnement réalisé afin d'éliminer les protéines résultant

de la réaction enzymatique et les réactifs n'ayant pas réagi. Ce fractionnement peut être réalisé, par exemple, par gel perméation selon le poids moléculaire et/ou la densité ionique des produits.

Dans un mode préféré de réalisation de l'invention, l'octasaccharide A—H est soumis à l'action d'une enzyme.

Comme enzyme appropriée pour obtenir l'élimination sélective des motifs AB de l'octasaccharide A—H, on a avantageusement recours à une héparinase, plus spécialement une héparinase d'origine bactérienne.

Une telle héparinase est avantageusement du type des héparinases pouvant être obtenues à partir des bactéries de Flavobacterium heparinum.

Pour une mise en oeuvre satisfaisante du procédé de l'invention d'obtention des hexasaccharides C'—H, on a plus spécialement recours à une héparinase telle qu'obtenue selon un procédé comportant des étapes, notamment de culture des bactéries de Flavobacterium heparinum d'extraction de l'héparinase brute à partir de ces bactéries, et des purifications, réalisées de manière à obtenir une héparinase purifiée, ayant une activité d'au moins 90 000 unités permettant d'effectuer l'élimination souhaitée des motifs A—B avec des rendements satisfaisants.

Avantageusement, l'héparinase mise en oeuvre possède une activité héparinasique supérieure à 100 000 unités, notamment, de l'ordre de 110 000 à 140 000 unités.

L'activité de l'enzyme est évaluée, par rapport à l'augmentation de l'absorption d'une héparine titrant au moins 215 µi/mg à 230 nm.

Par unités, on entend alors, dans la description et les revendications, la quantité d'enzymes qui entraîne l'apparition d'une augmentation d'un millième d'unité de densité optique par minute.

Comme déjà indiqué, on met en oeuvre de 0,25 à 1 mg d'enzyme (ayant une activité de l'ordre d'au moins 90 000 unités) par mg d'octasaccharide de préférence environ 0,5 mg d'enzyme.

L'étude de l'action de l'enzyme sur l'octasaccharide a montré qu'il était approprié d'opérer à une température supérieure à l'ambiante notamment entre 35 et 40° C, de préférence de l'ordre de 37° C.

Dans ces conditions, une durée totale d'environ 24 heures apparaît satisfaisante.

On opère avantageusement, en milieu tampon, de préférence à un pH de l'ordre de 6 à 8, en particulier voisin de la neutralité.

Compte tenu de la stabilité modérée de l'enzyme, il est préférable, afin d'augmenter son efficacité, de l'ajouter portions par portions, notamment à intervalles réguliers durant ce laps de temps.

On remarquera que, d'une manière avantageuse, le procédé de l'invention utilise un produit de départ, à savoir l'octasaccharide A—H de haute qualité: homogène, hautement spécifique.

Dans les conditions mises au point par les inventeurs, cet octasaccharide s'avère donc dégradable et ce, de manière sélective, en conduisant à des chaînes plus courtes conservant la séquence responsable de l'activité de ces produits et possédant donc avantageusement des propriétés biologiques au moins aussi importantes, voire supérieures à celles de l'octasaccharide.

De préférence, le procédé de l'invention est mis en oeuvre avec une héparinase obtenue par induction à partir de bactéries de Flavobacterium heparinum, extraction de l'héparinase brute à partir des bactéries, fractionnement de l'extrait brut d'héparinase obtenu suivi de plusieurs étapes de purifications des fractions possédant l'activité héparinasique recherchée.

La préparation d'héparinase s'effectue à partir d'une culture de Flavobacterium heparinum faite dans les conditions décrites par Payza et Coll. (J. Biol. Chem. 1956, 223, p. 853—858).

En opérant à une température voisine de l'ambiante, sous aération et agitation moyenne, une durée de culture de l'ordre de 25 à 30 heures apparaît satisfaisante.

Les bactéries sont alors récupérées du milieu de culture par exemple par centrifugation, de préférence réalisée à basse température, notamment inférieure à 10° C, de préférence de l'ordre de 4° C.

Avant d'extraire l'héparinase, il est avantageux de remettre en suspension les bactéries, puis après les avoir soumises à une opération de dispersion, de les lyophiliser.

Les bactéries sont alors soumises à un traitement en vue de l'extraction de l'héparinase. Ce traitement comprend avantageusement un broyage puis leur récupération par exemple par centrifugation.

Afin d'obtenir une extraction satisfaisante, il convient d'effectuer plusieurs broyages, par exemple, tout d'abord à sec puis en milieu tampon de pH de l'ordre de 6 à 8, avantageusement de 7 ou voisin de 7.

L'extrait brut d'héparinase récupéré par centrifugation est soumis, aux fins de purification, à une étape de fractionnement. Pour disposer d'une héparinase possédant l'activité et les propriétés recherchées, on procédé avantageusement à des opérations supplémentaires de purification des fractions successivement obtenues. L'étude de ces processus de fractionnement et de purification a montré qu'il était préférable d'opérer à une température inférieure à l'ambiante, en particulier inférieure à 10° C, de préférence de l'ordre de +4° C.

L'étape de fractionnement est avantageusement réalisée notamment selon un processus de chromatographie d'exclusion à l'aide de DEAE cellulose, en présence de sulfate d'ammonium.

Dans une première étape en batch, on utilise avantageusement la DEAE cellulose à raison d'environ au moins 3 g par g de cellules bactériennes, de préférence de l'ordre de 5 g.

La DEAE cellulose est avantageusement équilibrée au préalable à l'aide d'un tampon de pH de l'ordre de 6 à 8, de préférence de l'ordre de 7.

Le sulfate d'ammonium est mis en oeuvre à raison d'environ au moins 3 g/l, de préférence de l'ordre de 6 g/l.

La suspension ainsi formée est filtrée et le filtrat est recueilli, additionné avantageusement des solutions de rinçage de la DEAE cellulose. Dans une deuxième étape, la solution préalablement obtenue est soumise à un fractionnement supplémentaire à l'aide de DEAE cellulose, avantageusement prééquilibrée à l'aide du tampon utilisé précédemment. Cette étape est réalisée avec avantage par chromatographie dans une colonne, les filtrats étant percolés à un débit de l'ordre de 40 à 60 ml/h.

A partir de l'effluent, on récupère l'héparinase, par exemple, par précipitation, notamment à l'aide de sulfate d'ammonium.

L'héparinase obtenue à l'issue de cette opération de fractionnement, qui, dans les conditions particulières rapportées ci-dessus, se trouve sous forme d'un précipité sulfo-ammonique, est ensuite avantageusement purifiée selon un processus comportant au moins une mise en contact avec un agarose du type du Sépharose® plus spécialement celui connu sous la dénomination CM Sépharose CL 6 B® suivie d'une mise en contact des fractions purifiées, à activité héparinasique récueillies avec un agarose du type de celui commercialisé sous la dénomination ULTRAGEL ACA 54®.

L'étape de purification à l'aide d'un agarose du type du CM Sépharose CL 6 B® est avantageusement réalisée dans une colonne de chromatographie.

Il apparaît souhaitable de dissoudre dans de l'eau distillée l'héparinase (qui se trouve sous forme de précipité sulfoammonique), de manière à obtenir une solution ayant une conductivité de l'ordre de 6000 microhos et d'ajuster son pH à environ 6.

L'agarose mis en oeuvre est avantageusement équilibré au préalable à l'aide d'un tampon de pH de l'ordre de 5 à 7, de préférence voisin de 6.

Après avoir lavé la colonne de préférence à l'aide du tampon de lavage, on effectue la chromatographie de la solution d'héparinase et on récupère l'héparinase par élution par un gradient linéaire obtenu avec le tampon utilisé pour le lavage et ce même tampon amené à une force ionique plus élevée.

Les fractions possédant l'activité héparinasique recherchée sont récupérées pour recueillir l'héparinase qu'elles renferment. On effectue, par exemple, une précipitation notamment à l'aide de sulfate d'ammonium, suivie d'une centrifugation.

Comme déjà indiqué, il est avantageux de procéder à une purification supplémentaire de l'héparinase recueillie en opérant une nouvelle mise en contact avec un agarose, de préférence, un agarose tel que l'ULTROGEL ACA 54®. Cette opération est, de préférence, réalisée dans une colonne équilibrée à l'aide d'un tampon de pH de l'ordre de 6 à 8, notamment de l'ordre de 7.

Ce tampon est avantageusement utilisé pour développer la colonne.

On récupère ainsi des fractions à haute activité héparinasique.

Comme déjà décrit par les inventeurs, l'octasaccharide A—H mis en oeuvre dans le procédé de l'invention peut être obtenu par mise en contact de l'héparine (ou de fractions hépariniques) possédant une activité anticoagulante et des chaînes ayant un poids moléculaire de l'ordre de 2000 à 50 000 environ, avec un agent enzymatique, de préférence une héparinase purifiée, plus spécialement, d'origine bactérienne possédant une activité de l'ordre de 45 000 unités, étant entendu que le dosage est effectué avec une héparine titrant au moins 215 µi/mg. Les conditions utilisées pour la réalisation de cette étape sont ajustées de manière à obtenir un mélange de dépolymerisation contenant des chaînes octasaccharidiques ayant une activité anti-Xa (Yin-Wessler) et comprenant une séquence responsable de l'activité spécifique anti-Xa de ces produits.

L'enzyme provoque la coupure des chaînes hépariniques entre le carbone anomère du résidu N-sulfateglucosamine et du motif acide uronique suivant.

Les octasaccharides biologiquement actifs sont alors séparés du mélange de dépolymérisation par adsorption sur l'antithrombine III (ATIII) fixée sur un support tel que l'agarose, dans des conditions permettant aux octasaccharides ayant une affinité pour l'ATIII d'être fixés ou retenus sur l'ATIII.

Cette étape est avantageusement suivie de l'élution des produits retenus ou adsorbés afin de les récupérer et par leure fractionnement, par exemple par gel filtration afin de les isoler.

Ces oligosaccharides apparaissent capables d'exercer une activité antithrombotique puissante. En raison de leur activité anticoagulante faible et même pratiquement nulle, les risques d'hémorragie sont avantageusement pratiquement éliminés. On a de plus constaté que ce type d'oligosaccharides ne provoque aucune réactivité des plaquettes du sang.

Les hexasaccharides à chaînes courtes de l'invention sont dépourvus de toxicité. L'administration de 10 000 µ/kg (titre Yin-Wessler) de C'—H ne provoque chez le lapin aucune réaction toxique, ni d'effet pyrogénique dans le test de pyrogénicité chez le lapin conforme à la pharmacopée française.

L'administration à des souris de doses aussi importantes que 3200 mg/kg n'a pas permis de déterminer la $DL_{50}$.

L'invention est donc relative à des préparations pharmaceutiques qui renferment lesdits hexasaccharides à activité anti-Xa élevée.

Elle est plus particulièrement relative à des préparations pharmaceutiques dépourvues de substances pyrogènes contenant une quantité efficace de principes actifs en association avec des excipients pharmaceutiques.

Elle concerne également les compositions dans lesquelles le véhicule pharmaceutique est approprié pour l'administration par voie orale. Des formes d'administration de l'invention appropriées pour l'administration par voie orale peuvent être avantageusement des gélules pastrorésistantes, des comprimés ou tablettes, des pilules, ou encore présentées sous forme de liposomes.

D'autres compositions pharmaceutiques comprennent ces oligosaccharides en association avec les excipients appropriés pour l'administration par voie rectale. Des formes d'administration correspondantes sont constituées par des suppositoires.

D'autres formes d'aministration de l'invention sont constituées par des aérosols ou des pommades.

L'invention concerne également des compositions pharmaceutiques injectables, stériles ou stérilisables.

Ces solutions renferment avantageusement 1000 à 100 000 μ (Yin-Wessler)/ml d'oligosaccharides, de préférence de 5000 à 50 000, par exemple de 25 000 μ/ml, lorsque ces solutions sont destinées à l'injection par voie sous-cutanée. Elles peuvent contenir, par exemple, de 500 à 10 000, notamment 5000 μ/ml d'oligosaccharides lorsqu'elles sont destinées à l'injection par voie intraveineuse ou par perfusion.

Avantageusement, de telles préparation pharmaceutiques sont présentées sous la forme de seringues non récupérables, prêtes à l'emploi.

L'invention concerne également les compositions pharmaceutiques contenant lesdits oligosaccharides en association avec un autre principe actif, utilisable en particulier pour la prophylaxie et le traitement de thrombose, tel qu'un agent veinotonique comme la dihydroergotamine, un sel d'acide nicotinique ou un agent thrombolytique comme l'urokinase.

Les oligosaccharides à chaînes courtes de l'invention sont avantageusement sous la forme de sels d'au moins un métal physiologiquement acceptable tel que le sodium et/ou le calcium et/ou le magnésium.

Les compositions pharmaceutiques de l'invention sont particulièrement adaptées pour le contrôle (préventif ou curatif) de certaines étapes de la coagulation du sang chez l'homme ou l'animal), notamment dans le cas où le patient est soumis à des risques d'hypercoagulabilité résultant d'une perturbation en phase intrinsèque, par exemple comme conséquence d'une libération par l'organisme de thromboplastines, par exemple, de thromboplastines tissulaires (opérations chirurgicales, processus atheromateux, développement de tumeurs et troubles de la coagulation par des activateurs bactériens ou enzymatiques, etc).

Afin d'illustrer l'invention, on indique, ci-après, un exemple de posologie utilisable chez l'homme: cette posologie comprend, par exemple, l'administration au patient de 1000 à 25 000 μ (Yin et Wessler) par voie sous-cutanée, deux ou trois fois par jour, selon le niveau des risques d'hypercoagulabilité ou la condition thrombotique du patient, ou de 1000 à 25 000 μ/24 heures par voie intraveineuse, en administrations discontinues à intervalles réguliers, ou continues par perfusion, ou encore de 1000 à 25 000 μ (trois fois par semaine) par voie intramusculaire ou sous-cutanée (ces titres sont exprimés en unités Yin-Wessler). Ces doses peuvent être naturellement ajustée pour chaque patient en fonction des résultats et des analyses de sang effectuées auparavant, la nature des affections dont il souffre et, d'une manière générale, son état de santé.

L'invention se rapporte également à l'application des oligosaccharides de l'invention et des fractions qui les renferment, à la constitution de réactifs biologiques, utilisables en laboratoires, notamment comme éléments de comparaison pour l'étude d'autres substances dont on souhaite tester l'activité anticoagulante, notamment au niveau de l'inhibition du facteur Xa.

L'invention vise également les médicaments dans lesquels lesdits oligosaccharides sont marqués par des traceurs afin d'élaborer des produits radiopharmaceutiques utilisables en médecine nucléaire. On utilise les traceurs classiques, notamment, le technétium 99 m.

A cet effet, on transforme le technétium 99 m obtenu à partir de générateurs du commerce, sous forme de pertechnétate de sodium de valence 7 non réactif, en technétium réduit de valence 4 qui serait la forme la plus réactive du technétium. Cette transformation est effectuée grâce à un système réducteur réalisé à partir de sels détain (chlorure stanneux), de sels de fers (sulfate ferreux), de sels de titane (trichlorure de titane) ou autres sels.

La plupart du temps, cette simple réduction du technétium suffit dans des conditions de pH données, à réaliser la fixation du technétium sur la molécule considérée.

On peut utiliser les produits de l'invention, qui constituent en quelque sorte un support, à des doses de l'ordre de 100 à 200 μi Yin-Wessler.

Pour l'élaboration de ces réactifs radiopharmaceutiques, on peut opérer conformément à la méthode de P. V. KULKARNI et al. dans The Journal of Nuclear Medecine 21, N° 2, p. 117—121.

Les produits ainsi marqués sont avantageusement utilisés dans des tests in vivo pour la détection et le diagnostic d'extension des thromboses et des états thrombotiques.

Exemple 1

Procédé d'obtention des hexasaccharides actifs C'—H par action d'une héparinase sur l'octasaccharide A—H

Ce procédé comporte les trois étapes 1 à 3 suivantes:

1) la préparation de l'héparinase;
2) l'action de l'héparinase sur l'octasaccharide A—H aux fins de dégradation sélective, suivie par
3) le fractionnement du mélange de dégradation par filtration sur gel, et la récupération des fractions renfermant les hexasaccharides recherchés.

Ces étapes sont réalisées comme suit:

1) Préparation de l'héparinase

On utilise des enzymes provenant de la culture de flavobacterium heparinum obtenues en procédant comme suit:

Dans un fermenteur de 18 litres, du type de celui commercialisé sous la marque BIOLAFFITE®, on réalise la culture de flavobacterium heparinum ATCC 13 125, durant 26 heures dans un bouillon de culture répondant à la composition suivante (en grammes par litre d'eau distillée).

Bouillon de culture de Flavobacterium

| | |
|---|---|
| heparinum arrivé en phase stationnaire: | 500 ml |
| Phosphate de sodium monosodique: | 2,5 |
| Phosphate de sodium disodique: | 25 |
| Sulfate d'ammonium: | 1 |
| KCl: | 0,1 |

Héparinate de sodium de titre égal ou

| | |
|---|---|
| supérieur à 150 µi/mg qualité Codex: | 3 |
| Chlorure de calcium: | 0,01 |
| Chlorure ferrique: | 0,01 |
| Sulfate de magnésium: | 0,01 |
| Chlorure de manganèse: | 0,01 |
| Molybdate de sodium: | 0,01 |

Le pH du bouillon est finalement ajusté à 7,0 avec de l'acide phosphorique ou de la soude.

Cette culture est réalisée à une température de +24° C sous aération et agitation moyenne.

Après 26 heures de culture, le milieu est refroidi à +4° C en un laps de temps d'environ 2 heures. Les bactéries sont récupérées par centrifugation à 50 000 t/mn, sur une centrifugeuse du genre de la centrifugeuse SHARPLESS pneumatique type T 313 A® et ce durant 2 heures. Le culot de centrifugation est repris dans 1 litre d'eau distillée froide, soumis à une dispersion par turbine ULTRA-TURAX® à vitesse maximum pendant 5 minutes, puis lyophilisé. La durée totale de cette opération est d'environ 36 heures. Dans ces conditions, on obtient 4,1 grammes de cellules.

β — Extraction des cellules

Les cellules, lyophilisées, obtenues selon l'étape précédente, sont broyées vigoureusement à sec, au mortier, en présence de 2 g d'alumine calcinée, pendant 1 heure. On ajoute alors 10 ml de tampon 1) (tampon acétate de sodium 0,1 M, pH 7). Le broyage au mortier de la pâte alors obtenue est poursuivi pendant 30 minutes à +4° C. On ajoute ensuite 450 ml de tampon 1) froid et on laisse l'ensemble sous agitation pendant 1 heure à +4° C. La suspension obtenue est centrifugée sur une centrifugeuse du type SORVALL RC2 B®, à 18 000 t/mn à +4°C, pendant 20 minutes. Les culots de centrifugation composés d'alumine et de débris cellulaires sont écartés. On recueille le surnageant (jaune orangé et visqueux) qui constitue l'extrait cellulaire brut, et correspond à un volume de 470 ml. La suite des manipulations est effectuée à +4° C.

*γ* — Chromatographie d'exclusion par DEAE cellulose

Sous agitation, à +4°C, on ajoute 2,82 g de sulfate d'ammonium au surnageant précédemment obtenu, puis 21 g de DEAE cellulose préalablement équilibrée en tampon 2) (tampon acétate de sodium 0,1 M, pH 7 contenant 6 g/l de sulfate d'ammonium. On soumet l'ensemble à agitation durant 2 heures à +4°C avec contrôle et ajustement éventuel du pH 7,0. On sépare ensuite la DEAE cellulose par filtration sur büchner et on la lave avec du tampon 2) froid, jusqu'à absence de protéines dans les solutions de lavage. On passe l'ensemble filtrat et solutions de lavage (680 ml) sur une colonne de 400 ml (23=5 cm) de DEAE cellulose préequilibrée en tampon 2), à +4°C, à un débit de 50 ml/h. La colonne est finalement rincée par du tampon 2), jusqu'à absence de protéines dans les solutions de rinçages. On réunit les effluents de colonne et les solutions de rinçages, ce qui correspond à un volume de 1100 ml et on ajoute 715 g de sulfate d'ammonium, sous agitation. On recueille par centrifugation à 7000 t/mn les protéines précipitées et ce durant 30 minutes, sur une centrifugeuse du genre SORVALL®. On recueille 2,5 g de précipité très humide qui constitue l'héparinase. Ce précipité peut être stocké à —20°C durant plusieurs semaines.

*ε* — Chromatographie sur CM Sépharose CL 6 B®

On dissout le précipité sulfoammonique précédemment obtenu dans de l'eau distillée froide utilisée en quantité suffisante pour obtenir une conductivité finale de 6000 micromhos. On ajuste le pH de la solution obtenue à 6,0 par de l'acide acétique ou de la soude 2 N. Le volume final est de 440 ml. La solution est alors percolée à +4°C sur une colonne de 70 ml (15×2,6 cm) de CM Sépharose CL 6 B® préalablement équilibrée en tampon 3) (tampon acétate de sodium 0,1 M, NaCl 0,22 M, pH 6,0), à un débit de 25 ml/h. On rince la colonne par le tempon 3) jusqu'à absence de protéines dans l'effluent. Les effluents de colonne sont écartés.

L'héparinase est ensuite éluée à 60 ml/h à l'aide d'un gradient linéaire formé à partir de 600 ml de tampon 3) et de 600 ml de tampon 3) ajusté à 0,34 M de NaCl.

Les protéines sortant de la colonne sont détectées par enregistrement en continu de la densité optique (D.O.) à 280 nm et l'éluat est recueilli par fractions de 5 ml à l'aide d'un collecteur de fractions.

Sur la figure 1, on a représenté le graphique d'élution obtenu par enregistrement de la D.O. à 280 nm de l'effluent de colonne.

L'activité héparinasique de chaque fraction est dosée. On regroupe les fractions 45 à 48 (partie D du graphique) à haute teneur en héparinase. Ces fractions correspondent à un volume de 45 ml.

On précipite les protéines par addition de 30 g de sulfate d'ammonium et on les récupère par centrifugation à +4°C à 15 000 t/mn, durant 10 minutes.

— Gel filtration sur ULTROGEL ACA 54®

On dissout le culot de centrifugation précédemment obtenu dans de l'eau distillée froide et on obtient un volume final de 5 ml. Cette solution est déposée au sommet d'une colonne (1 m × 26 mm) d'ULTROGEL ACA 54® équilibrée en tampon acétate de sodium 0,1 M, NaCl 0,33 M, pH 7. La colonne est développée par ce même tampon à un débit de 15 ml par heure. Comme précédemment, les protéines sortant de la colonne sont détectées à 280 nm, et l'effluent de colonne est recueilli par fractions de 5 ml. Le graphique d'élution est représenté sur la figure 2. L'activité héparinasique de chaque fraction est dosée. Les fractions 32 à 37 (partie F du graphique), qui contiennent l'activité héparinasique, sont regroupées, ce qui correspond à un volume de 60 ml. Cette solution contient 7 mg d'héparinase purifiée, présentant une activité de l'ordre de 100 000 à 110 000 unités/mg. (On considère, comme unité d'enzyme, la quantité d'enzyme qui fait apparaître à 231 nm, un millième d'unité de densité optique par mn lorsqu'on met en contact à 38°C, de l'héparinase avec de l'héparine titrant au moins 215 µi/mg 0,065% en tampon tris-HCl, 0,125 M, pH 7, dans lequel on ajoute du chlorure de calcium $CaCl_2$).

La solution obtenue est conservée et congelée à —20°C.

2) Dégradation de l'octasaccharide par l'héparinase

On dissout 10 mg d'octasaccharide A—H (lot BC IV 135), obtenu selon le procédé décrit dans la demande de brevet EP 0 027 089 du 6. 10. 1980 au nom de la Demanderesse ayant une activité anti-Xa (Yin-Wessler) de 2100 µ/mg, dans 10 ml de tampon acétate de sodium 0,1 M de chlorure de calcium pH 7,2.

La solution est incubée à +37°C. L'addition d'héparinase est effectuée comme suit.

Au temps t=0, on ajoute 17 ml de la solution d'héparinase obtenue précédemment (soit 2 mg d'héparinase): au temps t1 =8 heures, on ajoute de nouveau 17 ml de solution d'héparinase: au temps

t2 = 16 heures, on ajoute finalement 8,5 ml de solution d'héparinase. Au temps t3 = 24 heures, on procède à l'évaporation à sec sous vide à 40°C, des 52,5 ml de solution sur un appareil du type Rotavapor BUCHI®.

3) Fractionnement du mélange de dégradation par filtration sur gel

On dépose le mélange obtenu à l'issue de l'étape de dégradation au sommet d'une colonne de Séphadex G-50 superfine® 200 x 2,5 cm. On procède à l'élution des produits à l'aide de chlorure de sodium 0,2 M. Les produits sont détectés par leur absorption à 230 nm. On rapporte sur la figure 3 le diagramme d'élution obtenu. On distingue 3 fractions principales. La première est constituée par du matériau de départ non dégradé (4 mg), la deuxième renferme les hexasaccharides recherchés (7 mg) et la troisième des disaccharides. On distingue également une fraction de moindre importance dans la région des tétrasaccharides entre les pics des hexa et des disaccharides.

On rassemble les fractions hexasaccharidiques, on élimine les sels et on les lyophilise.

— Etude de la structure des hexasaccharides des fractions recueillies

On procède à une étude de structure de ces fractions par analyse colorimétrique des fragments obtenus par dégradation à l'aide d'acide nitreux suivie d'une gel filtration. La dégradation, à l'aide d'acide nitreux, est effectuée selon la méthode de SHIVELY et CONRAD décrite dans Biochemistry, vol. 15, N° 12, 1976, p. 3932 à 3942.

L'action de l'acide nitreux se traduit par une coupure des liaisons glycosidiques entre les motifs N-sulfate-glucosamine et l'acide uronique suivant et transforme les motifs sulfate-glucosamine en résidus 2,5-anhydromannose.

L'hexasaccharide est alors transformé en tétrasaccharide et en disaccharide. On sépare ces deux oligosaccharides par filtration sur une colonne (200 x 0,6 cm) de Séphadex G-50 Superfine, éluée avec du chlorure de sodium 0,2 M. Sur la figure 4, on rapporte les valeurs de la densité optique mesurée à 230 nm, des fractions éluées (courbe a) en traits continus) ainsi que leur teneur en groupes 2,5-anhydromannose (courbe b) en pointillés . . .), acides uroniques (courbe c) en tirets — — —) et N-acétyl-glucosamine (courbe d) en tirets alternant avec des points —.—.—.) (pour la détermination de ce dernier groupe, on effectue des mesures avant et après hydrolyse acide, la différence obtenue correspondant à la teneur en groupes N-acétyl-glucosamine).

Il ressort de l'examen de la figure 4 (la flèche sur la figure 4 indique le volume d'élution pour l'hexasaccharide original) que les motifs acide uronique insaturé qui absorbent la lumière à 230 nm sont contenus dans la fraction tétrasaccharidique tandis que la fraction disaccharidique est pratiquement totalement dépourvue de tels composés.

De la même manière, il apparaît que les groupes N-acétyl-glucosamine sont seulement présents, comme prévu, dans la fraction tétrasaccharidique.

La teneur en groupe acide uronique apparaît deux fois plus importante dans la fraction tétrasaccharidique que dans la fraction disaccharidique.

On constate, par ailleurs, que les groupes 2,5-anhydromannose sont présents de manière équivalente dans les deux fractions, ce qui implique que les disaccharides et les tétrasaccharides sont obtenus dans un rapport molaire 1/1. Ces résultats montrent qu'une coupure a été opérée sur l'hexasaccharide, donnant lieu au disaccharide ne portant pas de double liaison et au tétrasaccharide (coupure entre F et G).

En outre, ces résultats établissent que la fraction hexasaccharidique est presque exclusivement constituée par une seule espèce qui porte un motif N-sulfate-glucosamine à son extrémité réductrice, un motif acide uronique insaturé à son extrémité non réductrice.

La comparaison avec la structure de l'octasaccharide de départ permet de conclure que deux autres motifs de glucosamine dont l'un est N-acétylé et l'autre N-sulfaté-3-O-sulfaté, et deux autres résidus d'acide uronique (un glucuronique et un iduronique-2-O-sulfaté) complètent la séquence hexasaccharide.

— Etude de l'activité biologique in vitro et in vivo de la fraction hexasaccharidique obtenue selon le procédé décrit ci-dessus

On détermine l'activité anti-Xa selon le test de Yin-Wessler décrit par ses auteurs dans J. Lab. Clin. Med. 1976, 81, 298—300.

L'activité anticoagulante globale est mesurée selon le test USP ou la méthode APTT.

Le test USP est décrit dans »Pharmacopea of the United States of America«, XIX, pages 229—230 (voir également le deuxième supplément USP-NF, page 62, et le quatrième supplément USP-NF, page 90, respectivement intitulés »Drug substances« et »Dosage forms«).

Le titre APTT est mesuré selon la méthode de J. CAEN et al. dans l'Hémostase, expansion scientifique française, Paris, 1968, pages 133—135.

L'activité antithrombotique in vivo est étudiée en utilisant la méthode de Wessler et al. décrite dans J. of. appl. physiol. 1959, 14, 943—946, en utilisant un stimulant thrombogénique différent.

— Activité anti-Xa (Yin-Wessler): 2400 µ/mg.
— Titre USP et titre APTT inférieurs à 10 µ/mg.

L'activité de cette fraction hexasaccharidique a été étudié in vivo sur le lapin selon le modèle de Wessler. L'administration de 250 µ anti-Xa par kg avant administration de 25 µ/kg d'un complexe thrombogénique (complexe cencentré de prothrombine vendu sous le nom de Konyn par Cutter Laboratories, U.S.A.), empêche la formation de thrombus.

**Revendications pour les Etats contractants: BE, CH, DE, GB, IT, LI, LU, NL, SE**

1. Compositions d'oligosaccharides à chaînes courtes, caractérisées en ce qu'elles sont formées essentiellement d'hexasaccharides possédant la séquence désignée ci-après par l'abréviation C'—H:

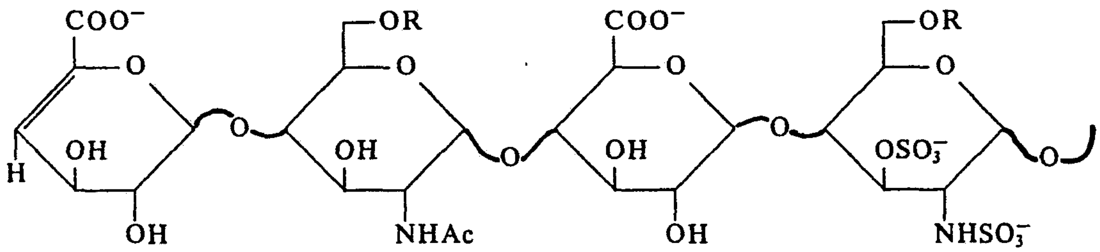

C' D E F

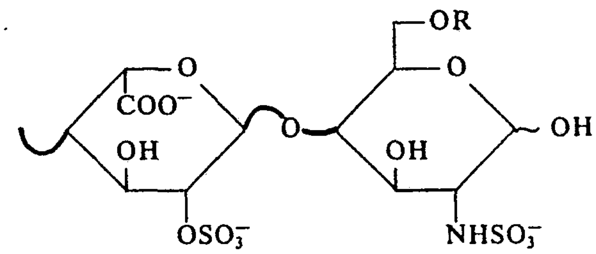

G H

dans laquelle représente un atome d'hydrogène ou le groupe $-SO_3^-$ et leurs sels physiologiquement acceptables.

2. Compositions selon la revendication 1, caractérisées en ce que lesdits hexasaccharides possèdent des titres Yin-Wessler supérieures à 2000 µ/mg, avantageusement 2500 µ/mg.

3. Compositions selon la revendication 1ou 2, caractérisées par des titres APTT ou USP de l'ordre de 10.

4. Compositions selon la revendication 2 ou 3, caractérisées par des titres Yin-Wessler et des titres USP pu APTT dans un rapport de l'ordre de 200, voire de 250 environ.

5. Compositions selon l'une quelconque des revendications précédentes, caractérisées en ce qu'elles sont formées d'hexasaccharides C'—H dans lesquels au moins un ou deux des groupes R représente un groupe $-SO_3^-$.

6. Procédé de préparation de compositions selon l'une quelconque des revendications précédentes, caractérisé en ce qu'on met en contact des octasaccharides répondant à la séquence ABCDEFGH (désignée ci-après par l'abréviation A—H), de formule

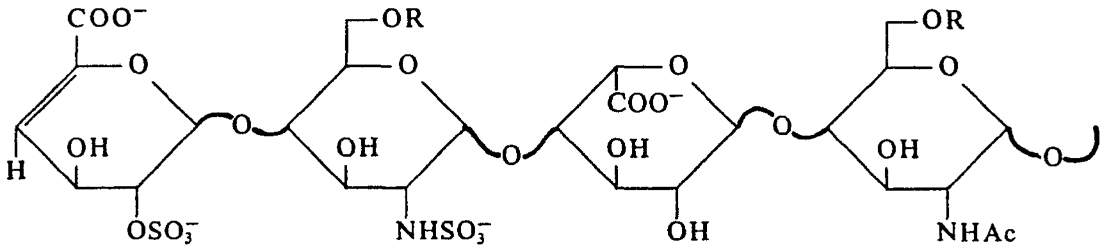

A B C D

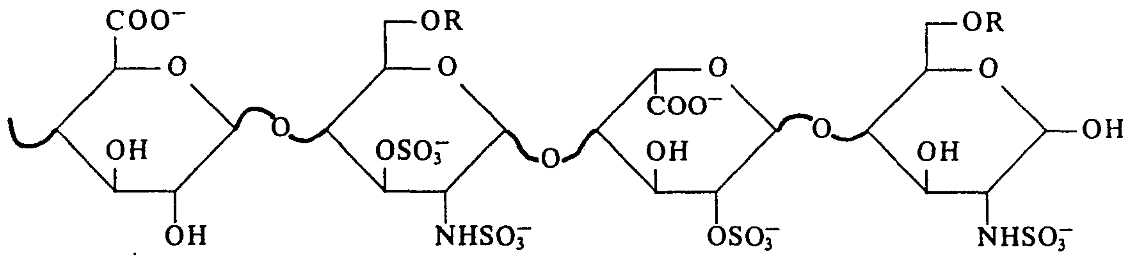

E F G H

ou des compositions octasaccharidiques formées d'une majeure partie de ces octasaccharides, ayant un rapport élevé de leur titre Yin-Wessler à leur titre APTT ou USP, avec un agent enzymatique présentant une activité enzymatique d'au moins environ 90 000 unités, mis en oeuvre à raison de 0,25 à 1 mg par mg d'octasaccharides traités, de préférence de l'ordre de 0,5 mg d'enzyme par mg d'octasaccharides, ce qui permet de fragmenter de manière spécifique les octasaccharides afin d'éliminer les motifs A—B, puis on sépare au moins la majeure partie des hexasaccharides C'—H actifs du mélange de dégradation.

7. Procédé selon la revendication 6, caractérisé en ce qu'on met en oeuvre comme enzyme, une héparinase, plus spécialement une héparinase d'origine bactérienne telle que celle pouvant être obtenue à partir des bactéries de Flavobacterium heparinum et qu'on utilise un milieu tampon renfermant du calcium.

8. Procédé selon la revendication 7, caractérisé en ce que l'héparinase mise en oeuvre possède une activité héparinasique de l'ordre de 110 000 à 140 000 unités (le dosage étant effectué avec une héparine titrant au moins 215 μi/mg à 230 nm).

9. Procédé selon l'une quelconque des revendications précédentes, caractérisé en ce que, pour disposer de compositions hexasaccharidiques hautement homogènes en hexasaccharides C'—H, on soumet le mélange résultant de la réaction enzymatique, à un fractionnement par exemple par gel perméation selon le poids moléculaire et/ou la densité ionique du produit.

10. Procédé selon l'une quelconque des revendications 6 à 9, caractérisé en ce qu'on opère à une température supérieure à l'ambiante, notamment entre 35 et 40° C, de préférence de l'ordre de 37° C, en milieu tampon de préférence à un pH de l'ordre de 6 à 8, en particulier voisin de la neutralité.

11. Procédé selon l'une quelconque des revendications 6 à 10, caractérisé en ce qu'on ajoute l'enzyme portion par portion, notamment à intervalles réguliers.

12. Médicaments, caractérisés en ce qu'ils renferment une quantité efficace d'au moins une composition d'oligosaccharides selon l'une quelconque des revendications 1 à 5.

13. Médicaments selon la revendication 12, caractérisés en ce qu'ils se présentent sous des formes appropriées pour l'administration par voie orale, avantageusement sous forme de gélules gastrorésistantes, de comprimés ou tablettes, de pilules, ou encore sous forme de liposomes, ou encore sous forme appropriée pour l'administration par voie rectale, sous forme de suppositoires, ou bien sous forme d'aérosols ou de pommandes ou encore sous forme de compositions injectables, utilisables pour le traitement préventif ou curatif de thromboses.

14. Médicaments selon la revendication 13, caractérisés en ce qu'ils se présentent sous forme de compositions pharmaceutiques injectables renfermant de 1000 à 100 000 μ (Yin-Wessler) par ml d'hexasaccharides, de préférence de 5000 à 50 000, notamment de 25 000 μ/ml lorsque ces solutions sont destinées à l'injection par voie sous-cutanée et de 500 à 10 000, notamment de 5000 μ/ml d'hexasaccharides lors qu'elles sont destinées à l'injection par voie intraveineuse ou par perfusion.

15. Médicaments selon l'une quelconque des revendications 12 à 14, caractérisés en ce que lesdits oligosaccharides sont marqués par des traceurs.

**Revendications pour l'Etat contractant: AT**

1. Procédé de préparation de compositions d'oligosaccharides à chaînes courtes possédant la séquence désignée ci-après par l'abréviation C'—H:

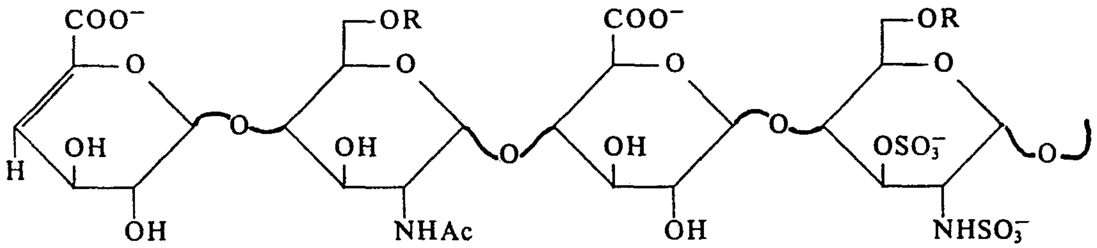

C' D E F

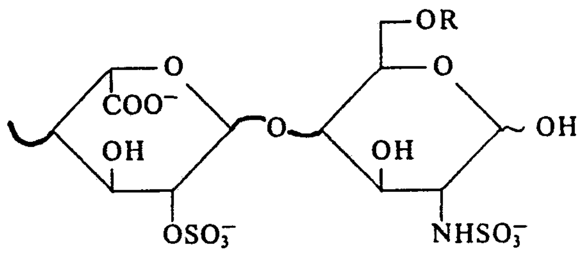

G H

dans laquelle R représente un atome d'hydrogène ou le groupe $-SO_3^-$ et leurs sels physiologiquement acceptables, caractérisé en ce qu'on soumet dans un milieu tampon renfermant du calcium, des octasaccharides répondant à la séquence ABCDEFGH désignée par l'abréviation A—H, de formule:

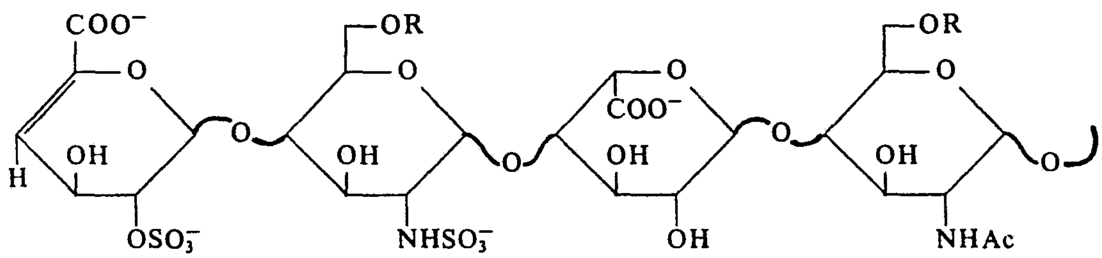

A B C D

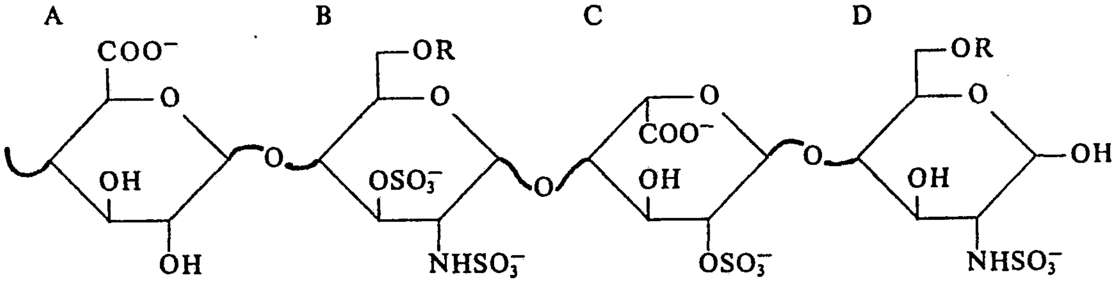

E F G H

ou des compositions octasaccharidiques formées d'une majeure partie de ces octasaccharides, ayant un rapport élevé de leur titre Yin-Wessler à leur titre APTT ou USP, à l'action d'un agent enzymatique, cet agent enzymatique présentant une acitivité enzymatique d'au moins environ 90 000 unités et étant mis en oeuvre à raison de 0,25 à 1 mg par mg d'octasaccharides traités, de préférence de l'ordre de 0,5 mg d'enzymes par mg d'octasaccharides, ce qui permet de fragmenter de manière spécifique les octasaccharides afin d'éliminer les motifs A—B, puis on sépare au moins la majeure partie des hexasaccharides C'—H actifs du mélange de dégradation.

2. Procédé selon la revendication 1, caractérisé en ce qu'on met en oeuvre comme enzyme, une héparinase, plus spécialement une héparinase d'origine bactérienne telle que celle pouvant être obtenue à partir des bactéries de Flavobacterium heparinum.

3. Procédé selon la revendication 2, caractérisé en ce que l'héparinase mise en oeuvre possède une activité héparinasique de l'ordre de 110 000 à 140 000 unités (le dosage étant effectué avec une

héparine titrant au moins 215 µi/mg à 230 nm).

4. Procédé selon l'une quelconque des revendications précédentes, caractérisé en ce que, pour disposer de compositions hexasaccharidiques hautement homogènes en hexasaccharides C'H, on soumet le mélange résultant de la réaction enzymatique, à un fractionnement par exemple par gel perméation selon le poids moléculaire et/ou la densité ionique du produit.

5. Procédé selon l'une quelconque des revendications 1 à 4, caractérisé en ce qu'on opère à une température supérieure à l'ambiante, notamment entre 35 et 40° C, de préférence de l'ordre de 37° C, en milieu tampon de préférence à un pH de l'ordre de 6 à 8, en particulier voisin de la neutralité.

6. Procédé selon l'une quelconque des revendications 1 à 5, caracterisé en ce qu'on ajoute l'enzyme portion par portion, notamment à intervalles réguliers.

7. Compositions d'oligosaccharides à chaînes courtes, caracterisées en ce qu'elles sont formées essentiellement d'hexasaccharides possédant la séquence désignée ci-après par l'abréviation C'—H:

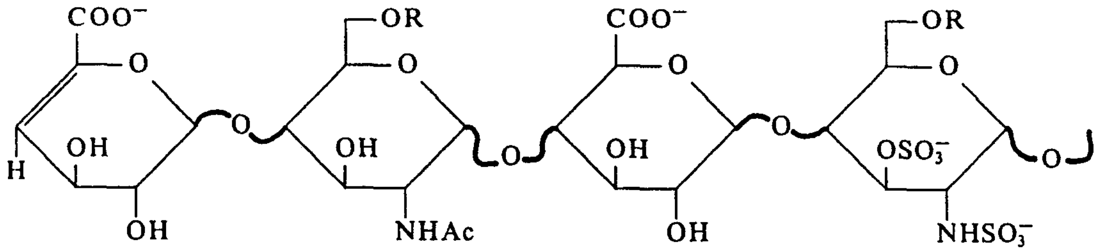

C' D E F

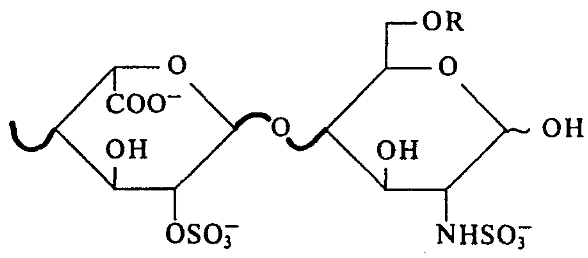

G H

dans laquelle R représente un atome d'hydrogène ou le groupe $-SO_3^-$ et leurs sels physiologiquement acceptables.

8. Compositions selon la revendications 7, caractérisées en ce que lesdits hexasaccharides possèdent des titres Yin-Wessler supérieure à 2000 µ/mg, avantageusement 2500 µ/mg.

9. Compositions selon la revendication 7 ou 8, caractérisées par des titres APTT ou USP de l'ordre de 10.

10. Compositions selon la revendication 8 ou 9, caractérisées par des titres Yin-Wessler et des titres USP ou APTT dans un rapport de l'ordre de 200, voire de 250 environ.

11. Compositions selon l'une quelconque des revendications 7 à 10, caractérisées en ce qu'elles sont formées d'hexasaccharides C'—H dans lesquels au moins un ou deux des groupes R représente un groupe $-SO_3^-$.

12. Médicaments, caractérisés en ce qu'ils renferment une quantité efficace d'au moins une composition d'oligosaccharides obtenue selon l'une quelconque des revendications 1 à 5.

13. Médicaments selon la revendication 12, caractérisés en ce qu'ils se présentent sous des formes appropriées pour l'administration par voie orale, avantageusement sous forme de gélules gastrorésistantes, de comprimés ou tablettes, de pilules, ou encore sous forme de liposomes, ou encore sous forme appropriée pour l'administration par voie rectale, sous forme de suppositoires, ou bien sous forme d'aérosols ou de pommades ou encore sous forme de compositions injectables, utilisables pour le traitement préventif ou curatif de thromboses.

14. Médicaments selon la revendications 13, caractérisés en ce qu'ils se présentent sous forme de compositions pharmaceutiques injectables renfermant de 1000 à 100 000 µ (Yin-Wessler) par ml d'hexasaccharides, de préférence de 5000 à 50 000, notamment de 25 000 µ/ml lorsque ces solutions sont destinées à l'injection par voie sous-cutanée et de 500 à 10 000, notamment de 5000 µ/ml d'hexasaccharides lorsqu'elles sont destinées à l'injection par voie intraveineuse ou par perfusion.

15. Médicaments selon l'une quelconque des revendications 12 à 14, caractérisés en ce que lesdits oligosaccharides sont marqués par des traceurs.

16. Procédé de préparation de médicaments, caractérisé en ce qu'on utilise une composition d'oligosaccharides selon la revendication 7 ou obtenue selon l'une quelconque des revendications 1 à 6 avec un excipient pharmaceutique.

**Patentansprüche für die Vertragsstaaten: BE, CH, DE, GB, IT, LI, LU, NL, SE**

1. Zusammensetzungen kurzkettiger Oligosaccharide, dadurch gekennzeichnet, daß sie im wesentlichen aus Hexasacchariden, welche die nachstehend mit der Abkürzung C'—H bezeichnete Sequenz:

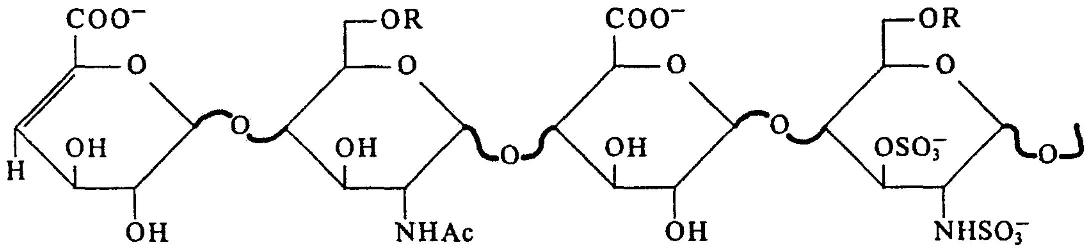

C' D E F

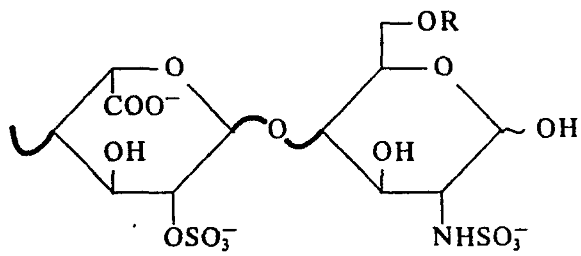

G H

aufweisen, in welcher R ein Wasserstoffatom oder die $-SO_3^-$-Gruppe bedeutet, und ihren physiologisch annehmbaren Salzen gebildet sind.

2. Zusammensetzungen nach Anspruch 1, dadurch gekennzeichnet, daß die genannten Hexasaccharide Titer nach Yin-Wessler aufweisen, die über 2000 Einheiten/mg, vorzugsweise bei 2500 Einheiten/mg, liegen.

3. Zusammensetzungen nach Anspruch 1 oder 2, gekennzeichnet durch APTT- oder USP-Titer in der Größenordnung von 10.

4. Zusammensetzung nach Anspruch 2 oder 3, gekennzeichnet durch Titer nach Yin-Wessler und USP- oder APTT-Titer in einem Verhältnis in der Größenordnung von 200, wie etwa 250.

5. Zusammensetzungen nach einem der vorhergehenden Ansprüche, dadurch gekennzeichnet, daß sie aus Hexasacchariden C'—H gebildet sind, in welchen wenigstens eine oder zwei der R-Gruppen eine $-SO_3^-$-Gruppe bedeuten.

6. Verfahren zur Herstellung von Zusammensetzungen nach einem der vorhergehenden Ansprüche, dadurch gekennzeichnet, daß man Octasaccharide, die der Sequenz ABCDEFGH (nachstehend mit der Abkürzung A—H bezeichnet) der Formel

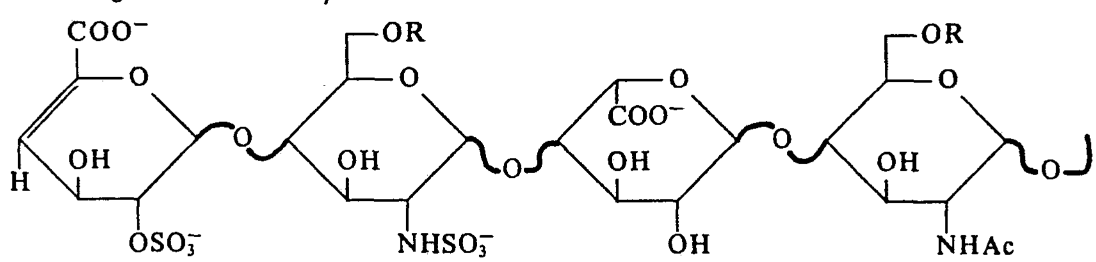

A B C D

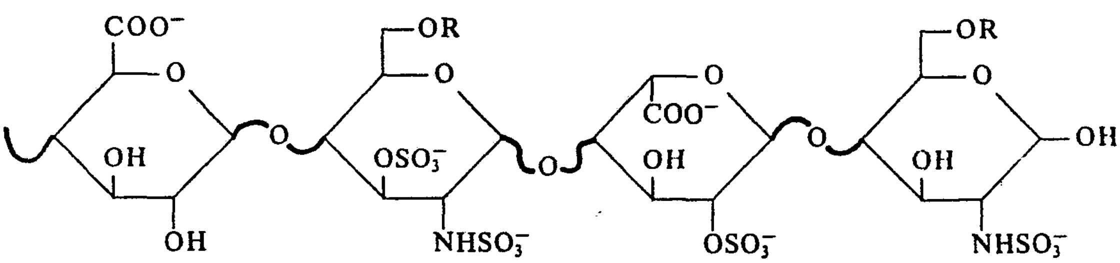

E F G H

entsprechen, oder Octasaccharidzusammensetzungen, die zu einem größeren Teil aus diesen Octasacchariden gebildet sind, mit einem hohen Verhältnis ihres Titers nach Yin-Wessler zu ihrem APTT- oder USP-Titer, mit einem enzymatischen Mittel in Berührung bringt, das eine enzymatische Aktivität von wenigstens etwa 90 000 Einheiten aufweist, und in einer Menge von 0,25 bis 1 mg je mg behandelter Octasaccharide, vorzugsweise in der Größenordnung von 0,5 mg Enzym je mg Octasaccharide, eingesetzt wird, was eine spezifische Spaltung der Octasaccharide unter Eliminierung der Abschnitte A—B gestattet, worauf man wenigstens den größeren Teil der aktiven Hexasaccharide C'—H aus dem Abbaugemisch abtrennt.

7. Verfahren nach Anspruch 6, dadurch gekennzeichnet, daß man als Enzym eine Heparinase, insbesondere eine Heparinase bakteriellen Ursprungs, wie eine solche, die ausgehend von Bakterien des Flavobacterium heparinum erhalten werden kann, einsetzt, und daß man ein calciumhältiges Puffermilieu anwendet.

8. Verfahren nach Anspruch 7, dadurch gekennzeichnet, daß die eingesetzte Heparinase eine Heparinaseaktivität in der Größenordnung von 110 000 bis 140 000 Einheiten besitzt (die Dosierung wird mit einem Heparintiter von wenigstens 215 iE/mg bei 230 nm bewirkt).

9. Verfahren nach einem der vorhergehenden Ansprüche, dadurch gekennzeichnet, daß man zur Gewinnung von Hexasaccharidzusammensetzungen hoher Homogenität bezüglich der Hexansaccharide C'—H die bei der enzymatischen Reaktion entstandene Mischung einer Fraktionierung, beispielsweise aufgrund von Geldurchlässigkeit in Abhängigkeit vom Molekulargewicht und/oder von der Ionendichte des Produktes, unterwirft.

10. Verfahren nach einem der Ansprüche 6 bis 9, dadurch gekennzeichnet, daß man bei einer oberhalb Umgebungstemperatur liegenden Temperatur, insbesondere zwischen 35 und 40°C, vorzugsweise in der Größenordnung von 37°C, in einem Puffermilieu, vorzugsweise bei einem pH im Bereich von 6 bis 8, insbesondere nahe der Neutralität, arbeitet.

11. Verfahren nach einem der Ansprüche 6 bis 10, dadurch gekennzeichnet, daß man das Enzym portionenweise, insbesondere in regelmäßigen Abständen, zugibt.

12. Arzneimittel, dadurch gekennzeichnet, daß sie eine wirksame Menge wenigstens einer Oligosaccharidzusammensetzung nach einem der Ansprüche 1 bis 5 enthalten.

13. Arzneimittel nach Anspruch 12, dadurch gekennzeichnet, daß sie in Formen vorliegen, die zur Verabreichung auf oralem Wege geeignet sind, vorteilhafterweise in Form von magensäureresistenten Geleepastillen, von Preßkörpern oder Tabletten, von Pillen oder auch in Form von Liposomen, oder auch in für die Verabreichung auf rektalem Wege entsprechender Form, in Form von Suppositorien, oder auch in Form von Aerosolen oder Salben, oder auch in Form von injizierbaren Zusammensetzungen, die zur vorbeugenden oder heilenden Behandlung von Thrombosen verwendbar sind.

14. Arzneimittel nach Anspruch 13, dadurch gekennzeichnet, daß sie in Form von injizierbaren, pharmazeutischen Zusammensetzungen vorliegen, die 1000 bis 100 000 Einheiten (Yin-Wessler) je ml Hexasaccharide, vorzugsweise 5000 bis 50 000, insbesondere 25 000 Einheiten/ml, enthalten, wenn diese Lösungen für eine subkutane Injektion vorgesehen sind, und die 500 bis 10 000, insbesondere 5000 Einheiten/ml, Hexasaccharide enthalten, falls sie für eine intravenöse Injektion oder für eine Perfusion vorgesehen sind.

15. Arzneimittel nach einem der Ansprüche 12 bis 14, dadurch gekennzeichnet, daß die genannten Oligosaccharide durch Tracer markiert sind.

**Patentansprüche für den Vertragsstaat: AT**

1. Verfahren zur Herstellung von Zusammensetzungen kurzkettiger Oligosaccharide, welche die nachstehend mit der Abkürzung C'—H bezeichnete Sequenz:

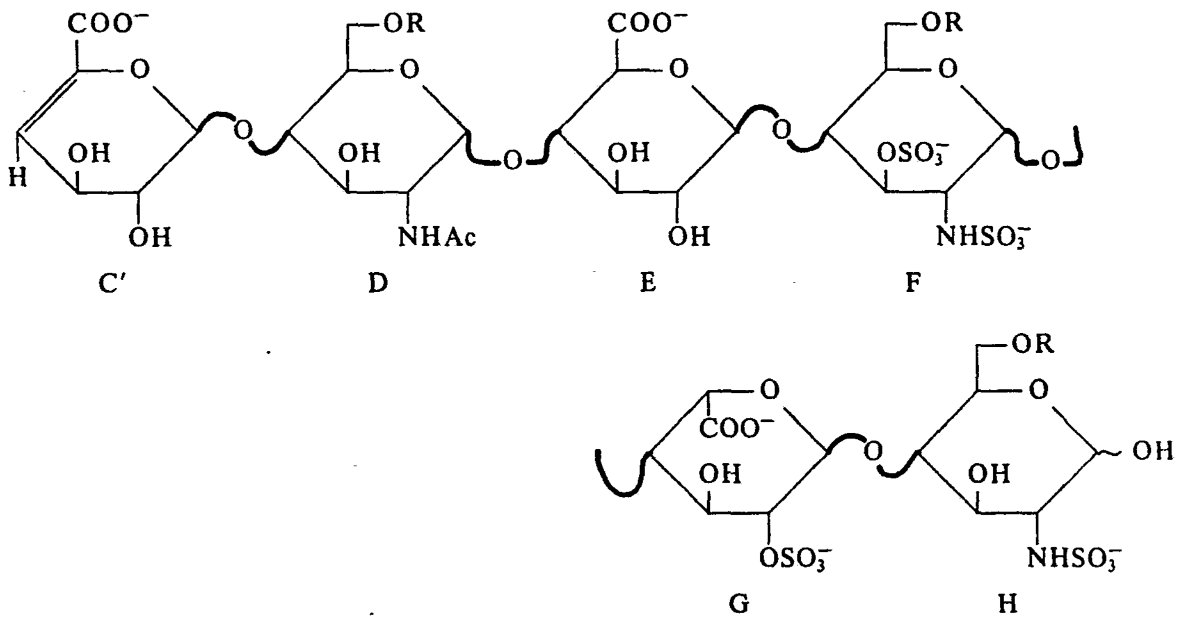

C' D E F

G H

aufweisen, in welcher R ein Wasserstoffatom oder die $-SO_3^-$-Gruppe bedeutet, und ihren physiologisch annehmbaren Salzen, dadurch gekennzeichnet, daß man Octasaccharide, die der mit der Abkürzung A—H bezeichneten Sequenz ABCDEFGH der Formel

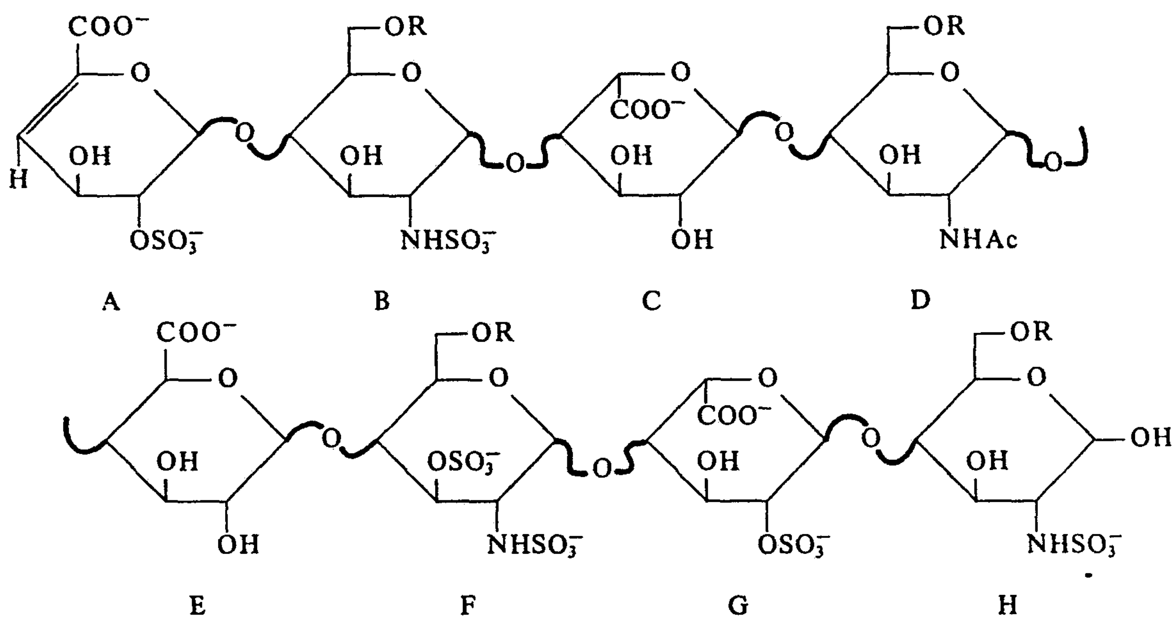

A B C D

E F G H

entsprechen, oder Octasaccharidzusammensetzungen, die zu einem größeren Teil aus diesen Octasacchariden gebildet sind, mit einem hohen Verhältnis ihres Titers nach Yin-Wessler zu ihrem APTT- oder USP-Titer, in einem calciumhältigen Puffermilieu der Einwirkung eines enzymatischen Mittels aussetzt, das eine enzymatische Aktivität von wenigstens etwa 90 000 Einheiten aufweist, und in einer Menge von 0,25 bis 1 mg je mg behandelter Octasaccharide, vorzugsweise in der Größenordnung von 0,5 mg Enzyme je mg Octasaccharide, eingesetzt wird, was eine spezifische Spaltung der Octasaccharide unter Eliminierung der Abschnitte A—B gestattet, worauf man wenigstens den größeren Teil der aktiven Hexasaccharide C'—H aus dem Abbaugemisch abtrennt.

2. Verfahren nach Anspruch 1, dadurch gekennzeichnet, daß man als Enzym eine Heparinase, insbesondere eine Heparinase bakteriellen Ursprungs, wie eine solche, die ausgehend von Bakterien des Flavobacterium heparinum erhalten werden kann, einsetzt.

3. Verfahren nach Anspruch 2, dadurch gekennzeichnet, daß die eingesetzte Heparinase eine

Heparinaseaktivität in der Größenordnung von 110 000 bis 140 000 Einheiten besitzt (die Dosierung wird mit einem Heparintiter von wenigstens 215 iE/mg bei 230 nm bewirkt).

4. Verfahren nach einem der vorhergehenden Ansprüche, dadurch gekennzeichnet, daß man zur Gewinnung von Hexasaccharidzusammensetzungen hoher Homogenität bezüglich der Hexasaccharide C'—H die bei der enzymatischen Reaktion entstandene Mischung einer Fraktionierung, beispielsweise aufgrund von Geldurchlässigkeit in Abhängigkeit vom Molekulargewicht und/oder von der Ionendichte des Produktes, unterwirft.

5. Verfahren nach einem der Ansprüche 1 bis 4, dadurch gekennzeichnet, daß man bei einer oberhalb Umgebungstemperatur liegenden Temperatur, insbesondere zwischen 35 und 40°C, vorzugsweise in der Größenordnung von 37°C, in einem Puffermilieu, vorzugsweise bei einem pH im Bereich von 6 bis 8, insbesondere nahe der Neutralität, arbeitet.

6. Verfahren nach einem der Ansprüche 1 bis 5, dadurch gekennzeichnet, daß man das Enzym portionenweise, insbesondere in regelmäßigen Abständen, zugibt.

7. Zusammensetzungen kurzkettiger Oligosaccharide, dadurch gekennzeichnet, daß sie im wesentlichen aus Hexasacchariden, welche die nachstehend mit der Abkürzung C'—H bezeichnete Sequenz:

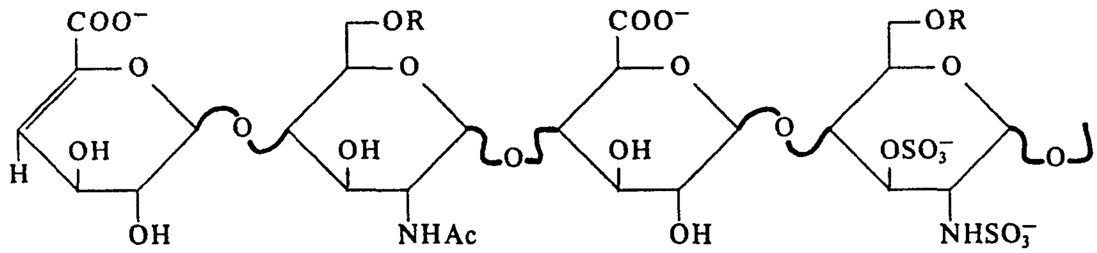

C' D E F

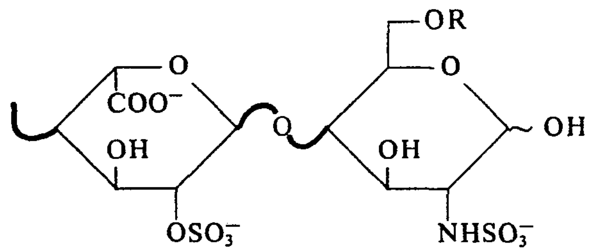

G H

aufweisen, in welcher R ein Wasserstoffatom oder die $-SO_3^-$-Gruppe bedeutet, und ihren physiologisch annehmbaren Salzen gebildet sind.

8. Zusammensetzungen nach Anspruch 7, dadurch gekennzeichnet, daß die genannten Hexasaccharide Titer nach Yin-Wessler aufweisen, die über 2000 Einheiten/mg, vorzugsweise bei 2500 Einheiten/mg, liegen.

9. Zusammensetzungen nach Anspruch 7 oder 8, gekennzeichnet durch APTT- oder USP-Titer in der Größenordnung von 10.

10. Zusammensetzungen nach Anspruch 8 oder 9, gekennzeichnet durch Titer nach Yin-Wessler und USP- oder APTT-Titer in einem Verhältnis in der Größenordnung von 200, wie etwa 250.

11. Zusammensetzungen nach einem der Ansprüche 7 bis 10, dadurch gekennzeichnet, daß sie aus Hexasacchariden C'—H gebildet sind, in welchen wenigstens eine oder zwei der R-Gruppen eine $-SO_3^-$-Gruppe bedeuten.

12. Arzneimittel, dadurch gekennzeichnet, daß sie eine wirksame Menge wenigstens einer nach einem der Ansprüche 1 bis 5 erhaltenen Oligosaccharidzusammensetzung enthalten.

13. Arzneimittel nach Anspruch 12, dadurch gekennzeichnet, daß sie in Formen vorliegen, die zur Verabreichung auf oralem Wege geeignet sind, vorteilhafterweise in Form von magensäureresistenten Geleepastillen, von Preßkörpern oder Tabletten, von Pillen oder auch in Form von Liposomen, oder auch in für die Verabreichung auf rektalem Wege entsprechender Form, in Form von Suppositorien, oder auch in Form von Aerosolen oder Salben, oder auch in Form von injizierbaren Zusammensetzungen, die zur vorbeugenden oder heilenden Behandlung von Thrombosen verwendbar sind.

14. Arzneimittel nach Anspruch 13, dadurch gekennzeichnet, daß sie in Form von injizierbaren, pharmazeutischen Zusammensetzungen vorliegen, die 1000 bis 100 000 Einheiten (Yin-Wessler) je ml Hexasaccharide, vorzugsweise 5000 bis 50 000, insbesondere 25 000 Einheiten/ml, enthalten, wenn diese Lösungen für eine subkutane Injektion vorgesehen sind, und die 500 bis 10 000, insbesondere 5000 Einheiten/ml, Hexasaccharide enthalten, falls sie für eine intravenöse Injektion oder für eine Perfusion vorgesehen sind.

15. Arzneimittel nach einem der Ansprüche 12 bis 14, dadurch gekennzeichnet, daß die genannten

Oligosaccharide durch Tracer markiert sind.

16. Verfahren zur Herstellung von Arzneimitteln, dadurch gekennzeichnet, daß man eine Oligosaccharidzusammensetzung nach Anspruch 7 oder eine nach einem der Ansprüche 1 bis 6 erhaltene Oligosaccharidzusammensetzung mit einem pharmazeutischen Träger verwendet.

**Claims for the Contracting states: BE, CH, DE, GB, IT, LI, LU, NL, SE**

1. Short chained oligosaccharide compositions, characterized in that they are formed essentially of hexasaccharides having the sequence denoted below by the abreviation C'—H:

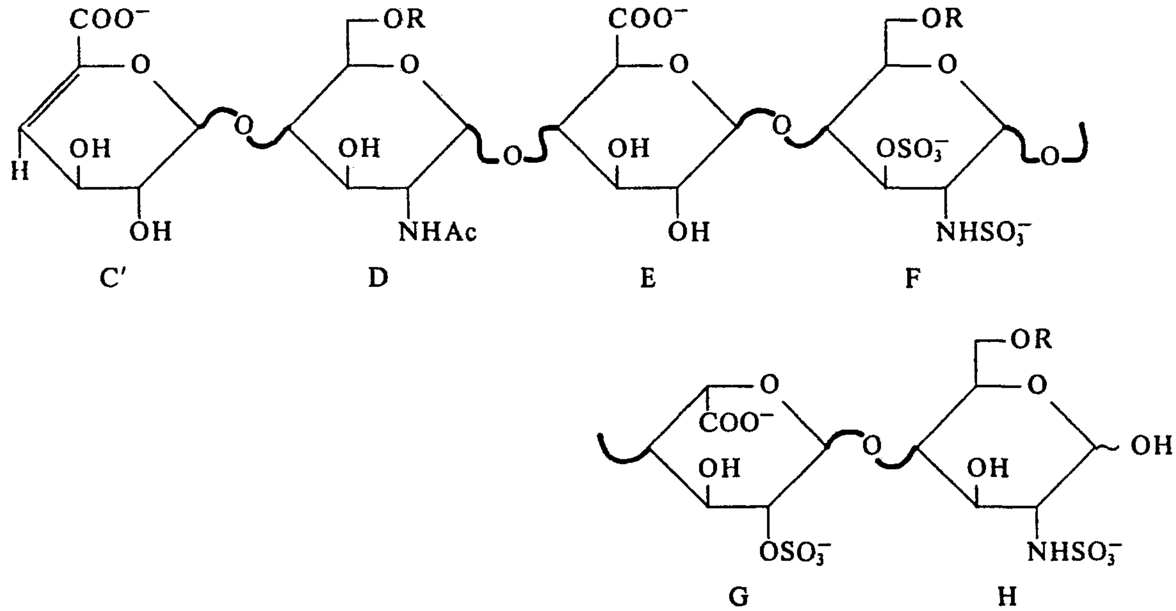

C' D E F

G H

in which R represents a hydrogen atom or the $-SO_3^-$ group and their physiologically acceptable salts

2. Compositions according to claim 1, characterized in that said hexasaccharides possess Yin-Wessler titers higher than 2000 µ/mg, advantageously 2500 µ/mg.

3. Compositions according to claim 1 or 2, characterized by APTT or USP titers of the order of 10.

4. Compositions according to claim 2 or 3, characterized by Yin-Wessler titers and USP or APTT titers in a ratio of the order of 200, even about 250.

5. Compositions according to anyone of the preceding claims, characterized in that they are formed of C'—H hexasaccharides in which at least one or two of the R groups represents a $-SO_3^-$ group.

6. A process for the preparation of compositions according to anyone of the preceding claims, characterized in that octasaccharides corresponding to the sequence ABCDEFGH (denoted below by the abreviation A—H) of the formula

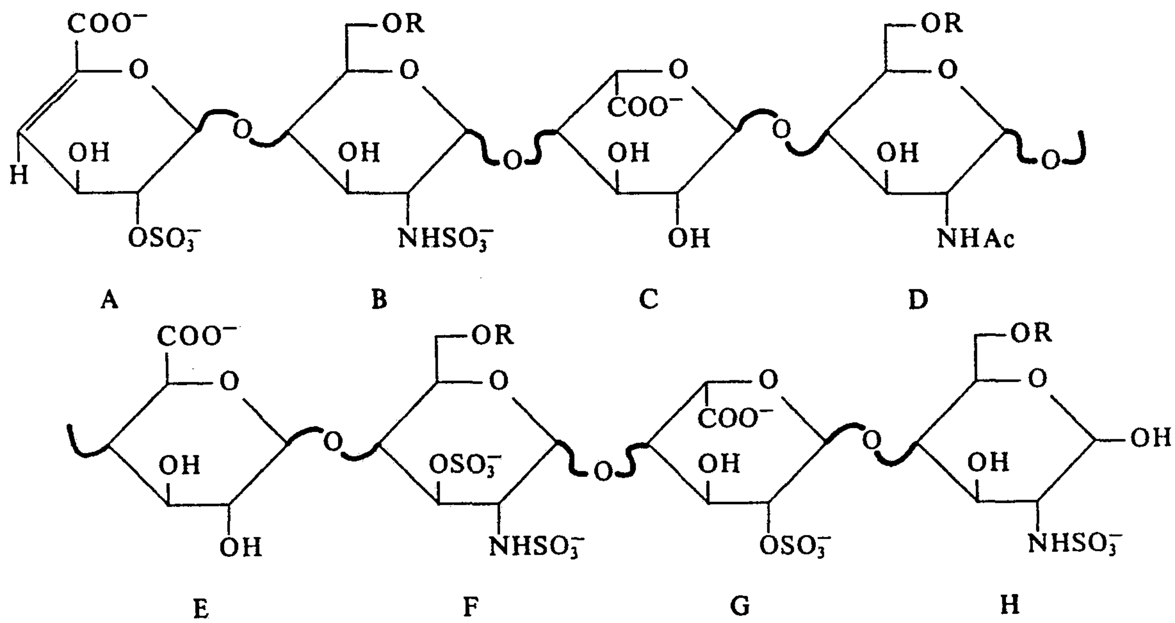

A B C D

E F G H

or octasaccharide compositions formed to a large extent of these octasaccharides, having a high ratio of their Yin-Wessler titer to their APTT or USP titer, are contacted with an enzymatic agent having an enzymatic activity of at least 90 000 units, applied at 0.25 to 1 mg per mg of octasaccharides treated, preferably of the order of 0.5 mg of enzymes per mg of octasaccharides, which enables to specifically fragment these octasaccharides in order to eliminroate A—B units and the major part of the active hexasaccharides C'—H are separated from the degradation mixture.

7. A process according to claim 6, characterized in that a heparinase is employed as enzyme, more especially a heparinase of bacterial origin such as that obtainable from Flavobacterium heparinum bacteria and a calcium containing buffer medium is used.

8. A process according to claim 7, characterized in that the heparinase applied possesses a heparinasic activity of about 110 000 to 140 000 units (the determination being effected with a heparin titrating at least 215 iμ/mg at 230 nm).

9. A process according to anyone of claims 6 to 8, characterized in that, to provide hexasaccharide compositions highly homogeneous in C'—H hexasaccharides, the mixture resulting from the enzymatic reaction is subjected to fractionation, for example, by gel permeation according to the molecular weight and/or the ionic density of the product.

10. A process according to anyone of claims 6 to 9, characterized in that the operation is carried out at a temperature higher than room temperature, particularly between 35 and 40°C, preferably of the order of 37°C, in a buffered medium preferably at a pH of the order of 6 to 8, in particular close to neutrality.

11. A process according to anyone of claims 6 to 10, characterized in that the enzyme is added portion by portion, particularly at regular intervals.

12. Drugs characterized in that they contain an effective amount of at least one composition of oligosaccharides according to anyone of claims 1 to 5.

13. Drugs according to claim 12, characterized in that they are in forms suitable for oral administration, advantageously in the form of gastroresistant capsules, compressed pellets or tablets, pills, or again in the form of liposomes, or again in suitable form for administration rectally, in the form of suppositories, or again in the form aerosols or of pomades, or again in the form of injectable compositions, useful for preventive or curative treatment of thromboses.

14. Drugs according to claim 13, characterized in that they are in the form of injectable pharmaceutical compositions containing from 1000 to 100 000 μ (Yin-Wessler) per ml of hexasaccharides, preferably from 5000 to 50 000, particularly 25 000 μ/ml when these solutions are intended for subcutaneous injection and from 500 to 10 000, particularly 5000 μ/ml of hexasaccharides when they are intended for intravenous injection or perfusion.

15. Drugs according to anyone of claims 12 to 14, characterized in that said oligosaccharides are labelled with tracers.

**Claims for the Contracting State: AT**

1. A process for the preparation of short chained oligosaccharide compositions, having the sequence denoted below by the abreviation C'—H:

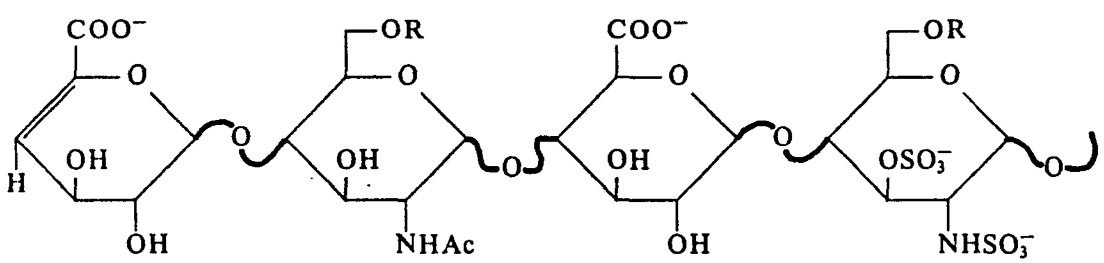

C' D E F

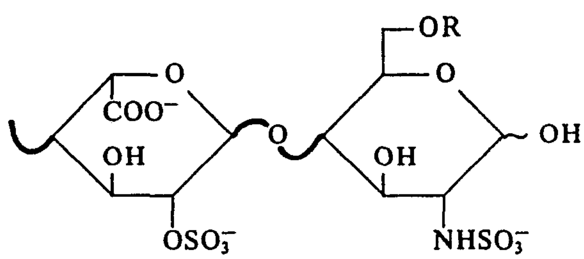

G H

in which R represents a hydrogen atom or the $-SO_3^-$ group and their physiologically acceptable salts, characterized in that octasaccharides corresponding to the sequence ABCDEFGH (denoted below by the abreviation A—H), of the formula

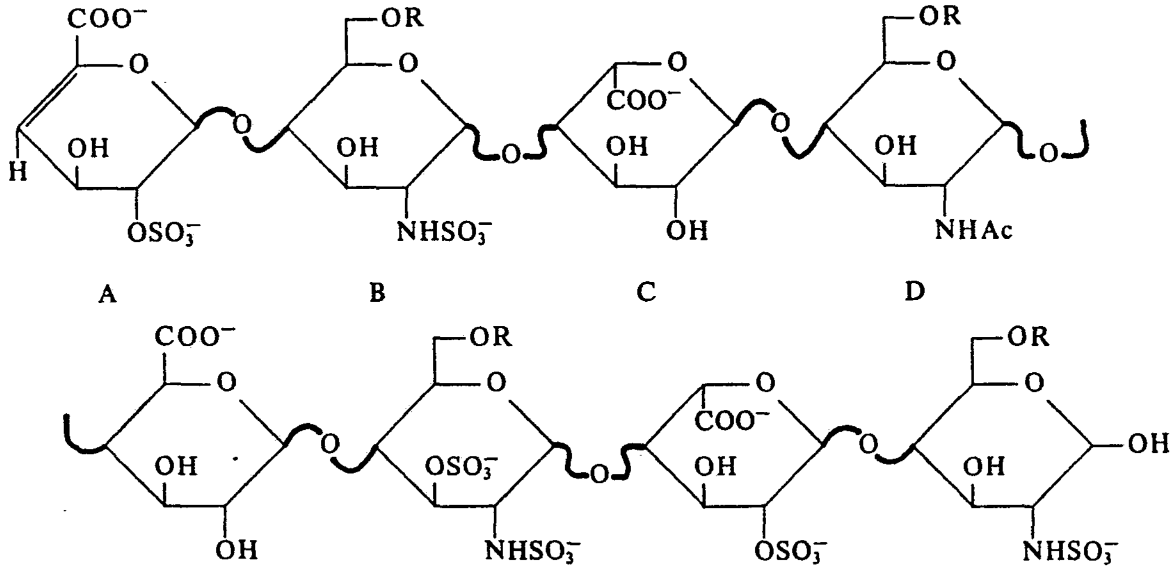

A B C D

E F G H

or octasaccharide compositions formed to a large extent of these octasaccharides, having a high ratio of their Yin-Wessler titer to their APTT or USP titer, are contacted, in a calcium containing buffer medium, with an enzymatic agent, said enzymatic agent having an enzymatic activity of at least 90 000 units and being applied at 0,25 to 1 mg per mg of octasaccharides treated, preferably of the order of 0.5 mg of enzymes per mg of octasaccharides, which enables to specifically fragment these octasaccharides in order to eliminate A—B units, and the major part of the active hexasaccharides C'—H are separated from the degradation mixture.

2. A process according to claim 1, characterized in that a heparinase is employed as enzyme, more especially a heparinase of bacterial origin such as that obtainable from Flavobacterium heparinum bacteria.

3. A process according to claim 2, characterized in that the heparinase applied possesses a heparinasic activity of about 110 000 to 140 000 units (the determination being effected with a heparin titrating at least 215 µi/mg at 230 nm).

4. A process according to anyone of the preceding claims, characterized in that, to provide hexasaccharide compositions highly homogeneous in C'—H hexasaccharides, the mixture resulting from the enzymatic reaction is subjected to fractionation, for example, by gel permeation according to the molecular weight and/or the ionic density of the product.

5. A process according to anyone of claims 1 to 4, characterized in that the operation is carried ou at a temperature higher than room temperature, particularly between 35 and 40°C, preferably of the order of 37°C, in a buffered medium preferably at a pH of the order of 6 to 8, in particular close to neutrality.

6. A process according to anyone of claims 1 to 5, characterized in that the enzyme is added portion by portion, particularly at regular intervals.

7. Short chained oligosaccharide compositions, characterized in that they are formed essentially of hexasaccharides having the sequence denoted below by the abreviation C'—H:

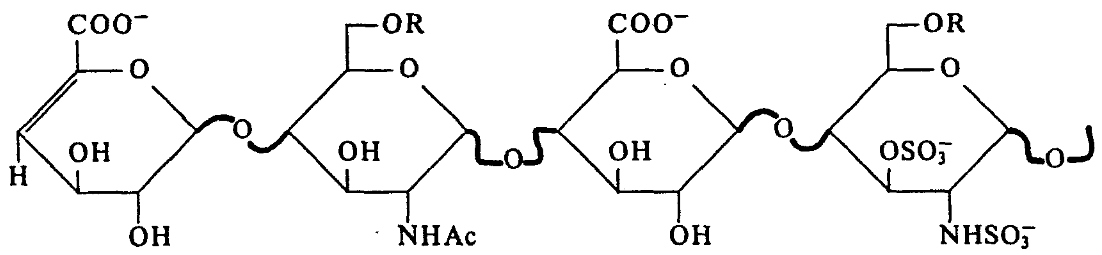

C' D E F

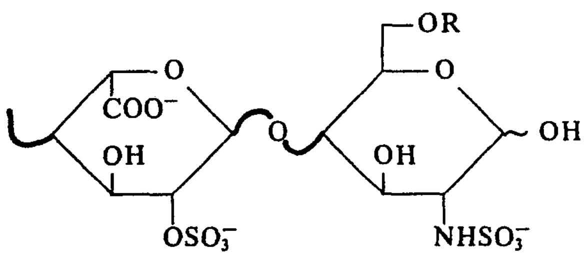

G H

in which R represents a hydrogen atom or the $-SO_3^-$ group and their physiologically acceptable salts.

8. Compositions according to claim 7, characterized in that said hexasaccharides possess Yin-Wessler titers higher than 2000 μ/mg advantageously 2500 μ/mg.

9. Compositions according to claim 7 or 8, characterized by APTT or USP titers of the order of 10.

10. Compositions according to claim 8 or 9, characterized by Yin-Wessler titers and USP or APTT titers in a ratio of the order of 200 even about 250.

11. Compositions according to anyone of claims 7 to 10, characterized in that they are formed of C'—H hexasaccharides in which at least one or two of the R groups represents a $-SO_3^-$ group.

12. Drugs characterized in that they contain an offective amount of at least one composition of oligosaccharides as obtained according to anyone of claims 1 to 5.

13. Drugs according to claim 12, characterized in that they are in forms suitable for oral administration, advantageously in the form of gastroresistant capsules, compressed pellets or tablets, pills, or again in the form of liposomes, or again in suitable form for administration rectally, in the form of suppositories, or again in the form aerosols or of pomades, or again in the form of injectable compositions, useful for preventive or curative treatment of thromboses.

14. Drugs according to claim 13, characterized in that they are in the form of injectable pharmaceutical compositions containing from 1000 to 100 000 μ (Yin-Wessler) per ml of Hexasaccharides, preferably from 5000 to 10 000, particularly 5000 μ/ml of hexasaccharides when they are intended for intravenous injection or perfusion.

15. Drugs according to anyone of claims 12 to 14, characterized in that said oligosaccharides are labelled with tracers.

16. A process for preparing drugs, comprising using an oligosaccharide composition according to claim 7 or obtained according to anyone of claims 1 to 6 with a pharmaceutical carrier.

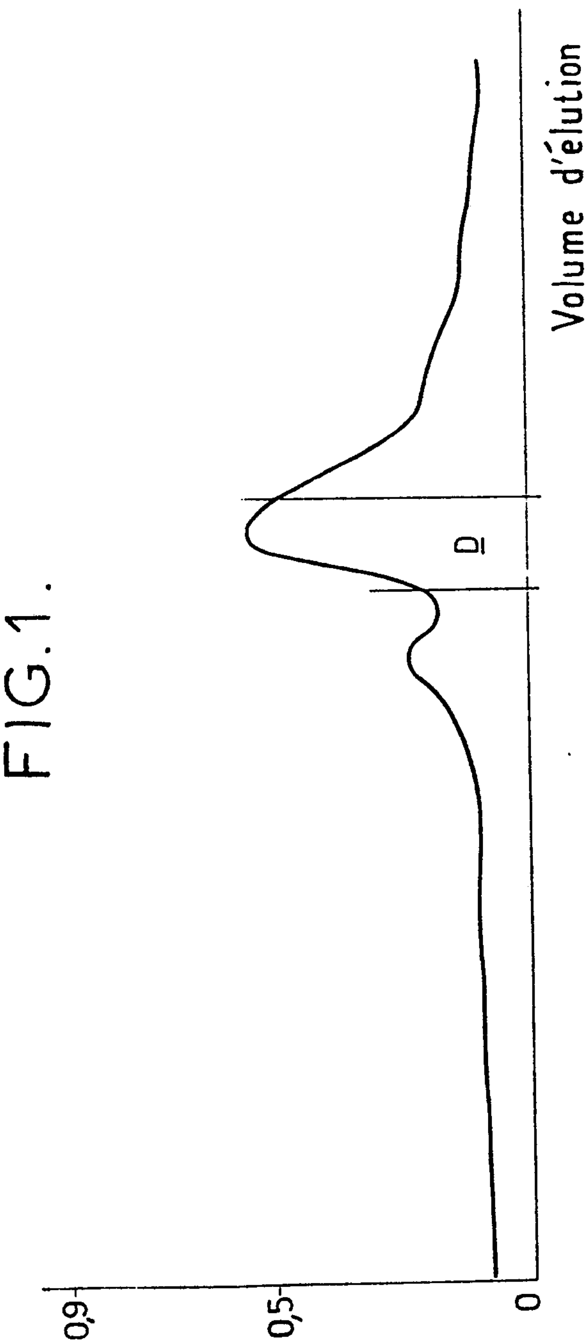


FIG.1.

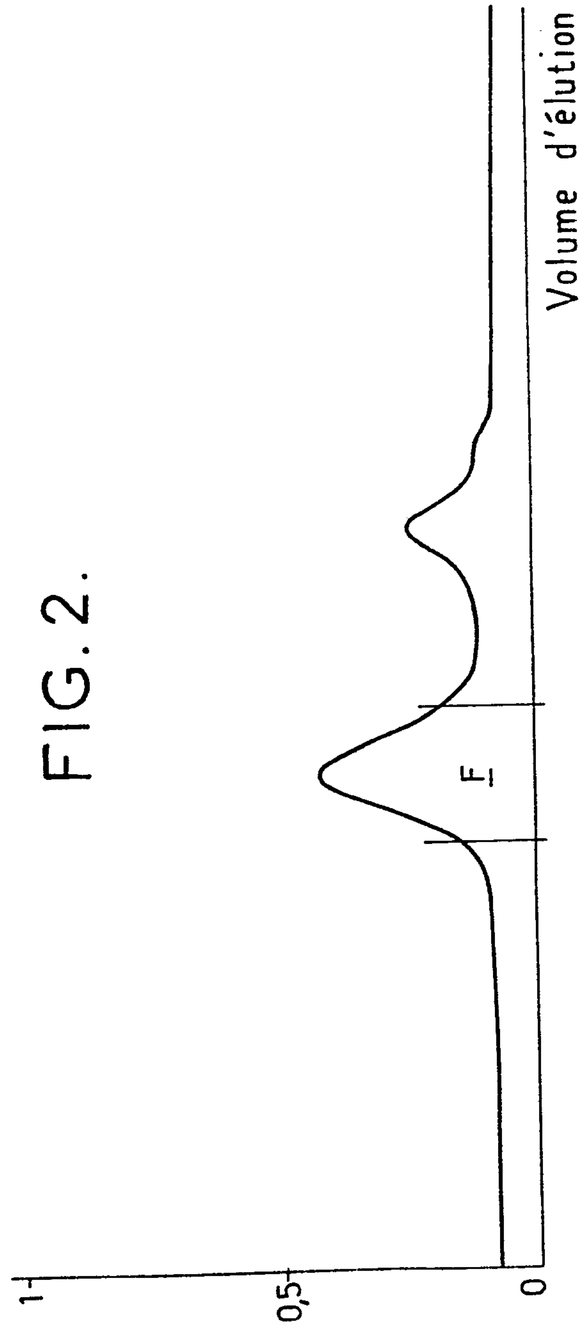


FIG.2.

FIG. 3.

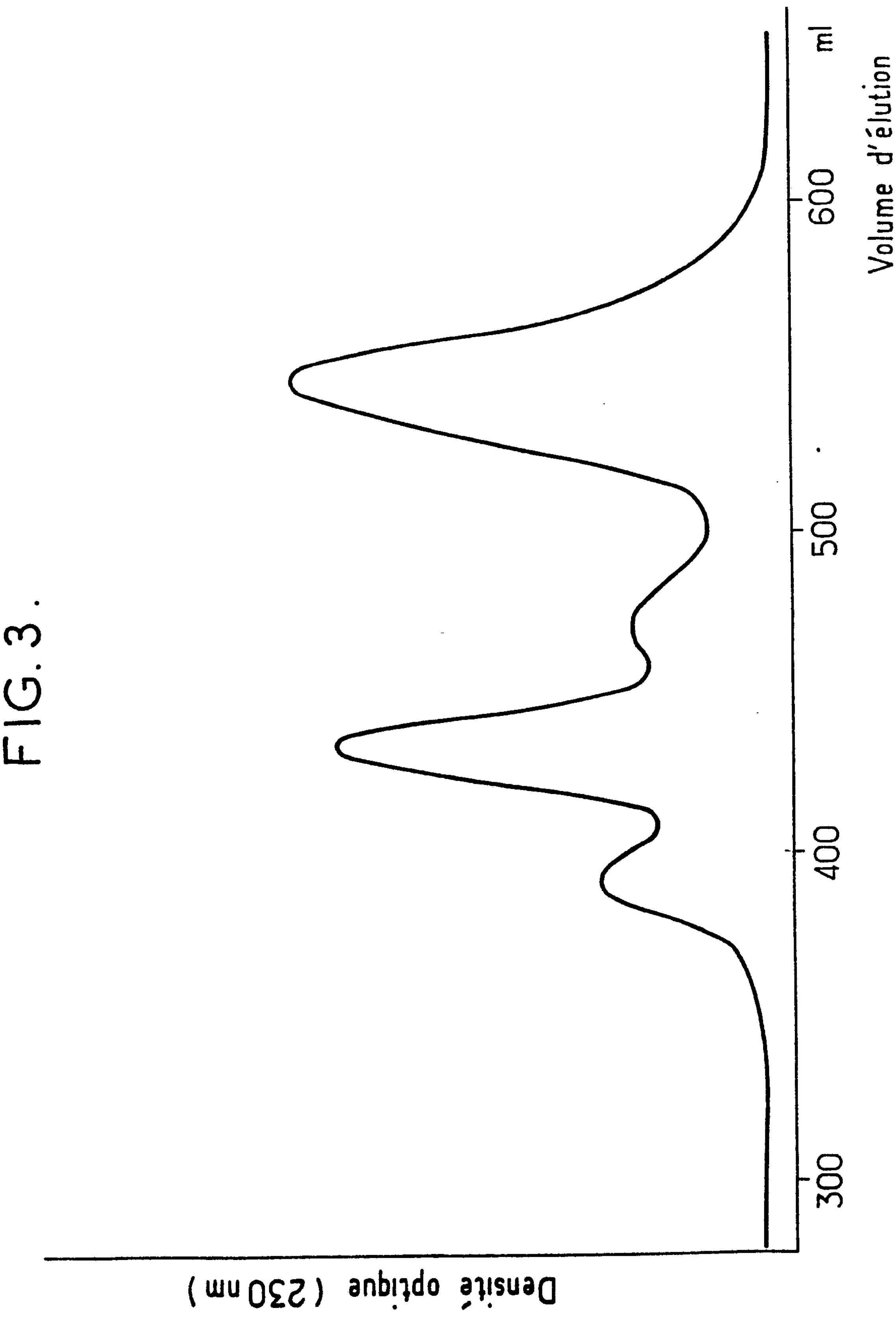

FIG. 4

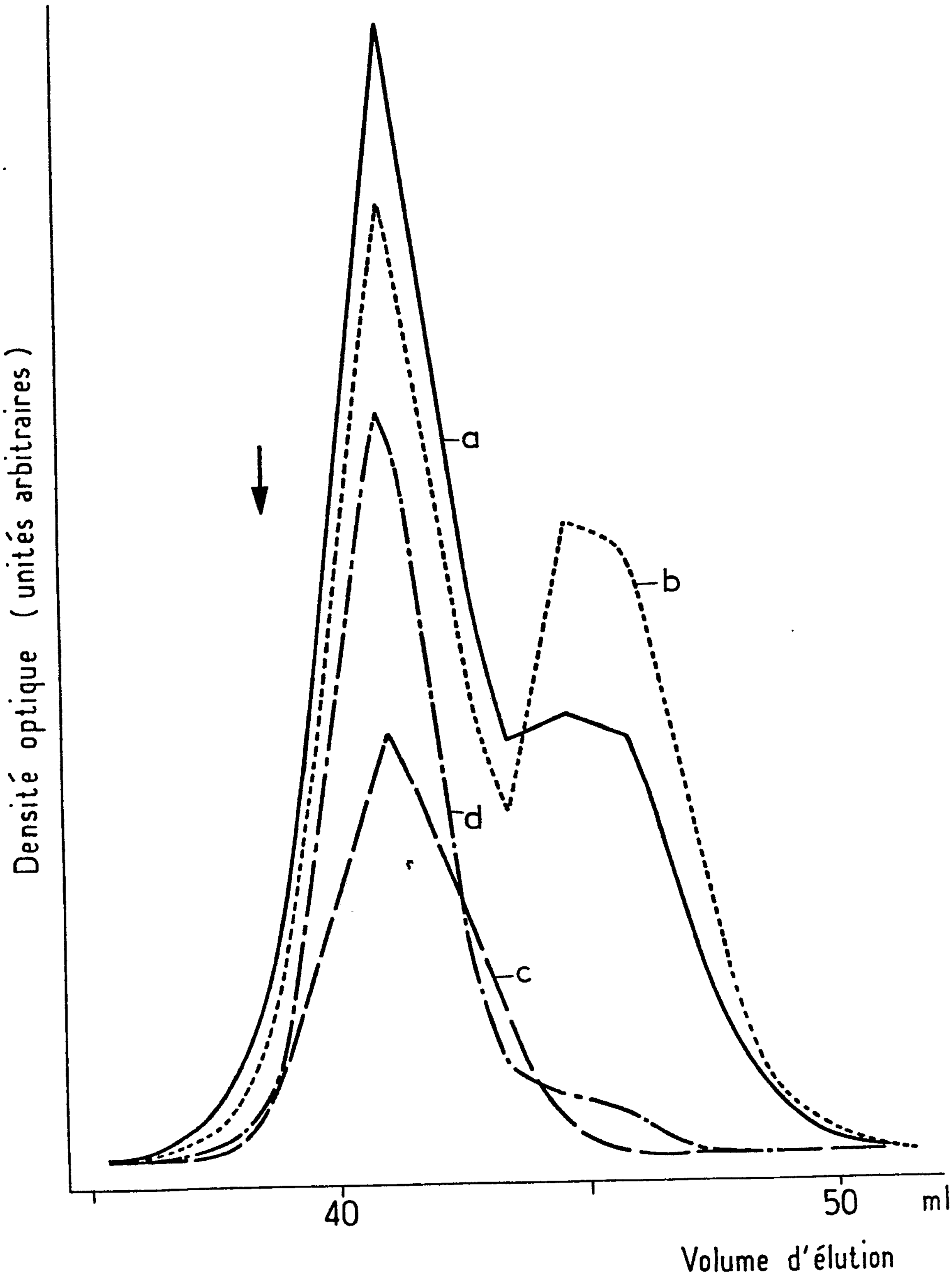
Densité optique (unités arbitraires)
a
b
c
d
40
50
ml
Volume d'élution